# EUROPEAN PATENT SPECIFICATION

(11) **EP 2 677 881 B1**
(45) Date of publication and mention of the grant of the patent: **01.04.2020**
(21) Application number: 12704836.1
(22) Date of filing: 22.02.2012
(51) Int. Cl.: C12N 9/16, C12N 9/20, A23K 10/30, A23K 20/189, A23K 10/14, A23K 50/10

(54) **FEED PROCESSING ENZYMES**
ENZYME ZUR FUTTERMITTEL BEHANDLUNG
ENZYMES DE TRAITEMENT D'ALIMENTS

(30) Priority: 24.02.2011 EP 11155883
(43) Date of publication of application: 01.01.2014
(73) Proprietor: Erber Aktiengesellschaft, 3130 Herzogenburg (AT)
(72) Inventor: BERLINGER, Marc, A-1050 Wien (AT); KALISZ, Henryk, A-1130 Wien (AT); MODREGGER, Jan, A-2345 Brunn am Gebirge (AT); SCHWAB-ANDICS, Christina, A-1090 Wien (AT); LUQUE, Alejandro, F-75017 Paris (FR); PANDJAITAN, Rudy, F-94700 Maisons Alfort (FR); HERRMANN, Manuela, A-8010 Graz (AT); REMLER, Peter, A-8010 Graz (AT)
(74) Representative: Cunow, Gerda
(86) International application number: PCT/EP2012/053009
(87) International publication number: WO 2012/113827

(56) References cited:
- WO-A1-01/23581
- WO-A1-96/12414
- DE-A1-102006 053 059
- US-A1- 2002 064 857
- US-A1- 2009 155 415
- US-A1- 2009 162 480
- TAKAHASHI-ANDO N ET AL: "A novel lactonohydrolase responsible for the detoxification of zearalenone: enzyme purification and gene cloning", BIOCHEMICAL JOURNAL, THE BIOCHEMICAL SOCIETY, LONDON, GB, vol. 365, 1 July 2002 (2002-07-01), pages 1-6, XP002967677, ISSN: 0264-6021, DOI: 10.1042/BJ20020450 cited in the application
- N. TAKAHASHI-ANDO ET AL: "Metabolism of Zearalenone by Genetically Modified Organisms Expressing the Detoxification Gene from Clonostachys rosea", APPLIED AND ENVIRONMENTAL MICROBIOLOGY, vol. 70, no. 6, 1 June 2004 (2004-06-01), pages 3239-3245, XP055005512, ISSN: 0099-2240, DOI: 10.1128/AEM.70.6.3239-3245.2004 cited in the application
- DATABASE WPI Week 201044 Thomson Scientific, London, GB; AN 2010-H36014 XP002657639, & CN 101 720 848 A (FEED RES INST CHINESE ACAD AGRIC SCI) 9 June 2010 (2010-06-09)
- MOLNAR O ET AL: "Trichosporon mycotoxinivorans sp. nov., A New Yeast Species Useful in Biological Detoxification of Various Mycotoxins", SYSTEMATIC AND APPLIED MICROBIOLOGY, URBAN UND FISCHER VERLAG, DE, vol. 27, no. 6, 15 December 2004 (2004-12-15), pages 661-671, XP004962913, ISSN: 0723-2020, DOI: 10.1078/0723202042369947
- EL-SHARKAWY S ET AL: "MICROBIAL CLEAVAGE OF ZEARALENONE", XENOBIOTICA, vol. 18, no. 4, 1988, pages 365-372, XP009151537, ISSN: 0049-8254
- SCHATZMAYR G ET AL: "Detoxification of mycotoxins by biotransformation", THE MYCOTOXIN FACTBOOK: FOOD AND FEED TOPICS, X, XX, 1 January 2006 (2006-01-01), pages 363-375, XP009151517, ISBN: 978-90-8686-006-7

### SEQUENCE LISTING

<110> Eucodis Bioscience GmbH
<120> FEED PROCESSING ENZYMES
<130> EU001P
<160> 16
<170> PatentIn version 3.5
<210> 1
   <211> 933
   <212> DNA
   <213> Artificial Sequence
<220>
   <223> nucleic acid sequence of triacylglycerol hydrolase
<400> 1
   atgccgcagc acctgctgcc ggctcgtcgt caggctgctc gtccgtctcg tccgcgtacc      60
   ctgaccggtc tgctggctgc tgctgctgct accgctggtc tgctgctgtc tggtctggct     120
   ccgggtgctc aggctgctgc tgctgctgct aacccgtacg aacgtggtcc ggctccgacc     180
   aacgcttcta tcgaggcttc tcgtggtccg tacgctacct ctcagacctc tgtttcttct     240
   ctggttgctt ctggtttcgg tggtggtacc atctactacc cgacctctac cgctgacggt     300
   accttcggtg ctgttgttat ctctccgggt ttcaccgctt accagtcttc tatcgcttgg     360
   ctgggtccgc gtctggcttc tcagggtttc gttgttttca ccatcgacac caacaccacc     420
   ctggaccagc cggactctcg tggtcgtcag ctgctgtctg ctctggacta cctgacccag     480
   cgttcttctg ttcgtacccg tgttgacgct acccgtctgg gtgttatggg tcactctatg     540
   ggtggtggtg gttctctgga agctgctaaa tctcgtacct ctctgaaagc tgctatcccg     600
   ctgaccggtt ggaacaccga caaaacctgg ccggaactgc gtaccccgac cctggttgtt     660
   ggtgctgacg gtgacaccgt tgctccggtt gctacccact ctaaaccgtt ctacgaatct     720
   ctgccgggtt ctctggacaa agctcacctg gaactgcgtg gtgcttctca cttcaccccg     780
   aacacctctg acaccaccat cgctaaatac tctatctctt ggctgaaacg tttcatcgac     840
   tctgacaccc gttacgaaca gttcctgtgc ccgatcccgc gtccgtctct gaccatcgct     900
   gaataccgtg gtacctgccc gcacacctct tga                                  933
<210> 2
   <211> 310
   <212> PRT
   <213> Artificial Sequence
<220>
   <223> amino acid seqence of triacylglycerol hydrolase
<400> 2
   Met Pro Gln His Leu Leu Pro Ala Arg Arg Gln Ala Ala Arg Pro Ser
   1               5                   10                  15
   Arg Pro Arg Thr Leu Thr Gly Leu Leu Ala Ala Ala Ala Ala Thr Ala
               20                  25                  30
   Gly Leu Leu Leu Ser Gly Leu Ala Pro Gly Ala Gln Ala Ala Ala Ala
           35                  40                  45
   Ala Ala Asn Pro Tyr Glu Arg Gly Pro Ala Pro Thr Asn Ala Ser Ile
       50                  55                  60
   Glu Ala Ser Arg Gly Pro Tyr Ala Thr Ser Gln Thr Ser Val Ser Ser
   65                  70                  75                      80
   Leu Val Ala Ser Gly Phe Gly Gly Gly Thr Ile Tyr Tyr Pro Thr Ser
                   85                  90                  95
   Thr Ala Asp Gly Thr Phe Gly Ala Val Val Ile Ser Pro Gly Phe Thr
               100                 105                 110
   Ala Tyr Gln Ser Ser Ile Ala Trp Leu Gly Pro Arg Leu Ala Ser Gln
           115                 120                 125
   Gly Phe Val Val Phe Thr Ile Asp Thr Asn Thr Thr Leu Asp Gln Pro
       130                 135                 140
   Asp Ser Arg Gly Arg Gln Leu Leu Ser Ala Leu Asp Tyr Leu Thr Gln
   145                 150                 155                     160
   Arg Ser Ser Val Arg Thr Arg Val Asp Ala Thr Arg Leu Gly Val Met
                   165                 170                 175
   Gly His Ser Met Gly Gly Gly Gly Ser Leu Glu Ala Ala Lys Ser Arg
               180                 185                 190
   Thr Ser Leu Lys Ala Ala Ile Pro Leu Thr Gly Trp Asn Thr Asp Lys
           195                 200                 205
   Thr Trp Pro Glu Leu Arg Thr Pro Thr Leu Val Val Gly Ala Asp Gly
       210                 215                 220
   Asp Thr Val Ala Pro Val Ala Thr His Ser Lys Pro Phe Tyr Glu Ser
   225                 230                 235                     240
   Leu Pro Gly Ser Leu Asp Lys Ala His Leu Glu Leu Arg Gly Ala Ser
                   245                 250                 255
   His Phe Thr Pro Asn Thr Ser Asp Thr Thr Ile Ala Lys Tyr Ser Ile
   260 265 270
   Ser Trp Leu Lys Arg Phe Ile Asp Ser Asp Thr Arg Tyr Glu Gln Phe
   275 280 285
   Leu Cys Pro Ile Pro Arg Pro Ser Leu Thr Ile Ala Glu Tyr Arg Gly
   290 295 300
   Thr Cys Pro His Thr Ser
   305 310
<210> 3
   <211> 795
   <212> DNA
   <213> Artificial Sequence
<220>
   <223> nucleic acid sequence of zearalenone hydrolase
<400> 3
   atgcgcactc gcagcacaat ctcgaccccg aatggcatca cctggtacta tgagcaggag 60
   ggtactggac ccgacgttgt cctcgttccc gatggcctcg gagaatgcca gatgtttgac 120
   agctccgtgt cgcagattgc tgctcaaggc ttcagggtca ctacgtttga tatgcccgga 180
   atgtcccggt ctgccaaggc accacccgag acctacactg aggtcacggc ccagaagctg 240
   gcttcctatg tcatctccgt cctggatgct cttgacatca agcacgctac tgtctggggc 300
   tgcagctcag gagcttccac cgtcgtggcg ctgttgctcg gttaccccga caggatacgc 360
   aacgccatgt gccacgaact gccaacaaag ctactggacc acctttcaaa caccgctgtg 420
   ctcgaagacg aggaaatctc aaagatcctg gccaatgtaa tgttgaacga cgtgtctgga 480
   ggctcggagg cgtggcaagc catgggggac gaggtgcacg cgagactgca caagaactac 540
   ccggtttggg ctcgaggata ccctcggact attcctccct cagctccggt taaggatctg 600
   gaggctctgc gtgggaagcc cctggactgg actgtcggcg ctgcgacacc aaccgagtct 660
   ttctttgaca acattgttac cgctaccaag gctggtgtca acattgggtt gcttccaggg 720
   atgcatttcc cttatgtttc ccacccggac gttttcgcta aatatgttgt ggaaactacg 780
   cagaagcatc tttga 795
<210> 4
   <211> 264
   <212> PRT
   <213> Artificial Sequence
<220>
   <223> amino acid sequence of zearalenone hydrolase
<400> 4
   Met Arg Thr Arg Ser Thr Ile Ser Thr Pro Asn Gly Ile Thr Trp Tyr
   1               5                   10                  15
   Tyr Glu Gln Glu Gly Thr Gly Pro Asp Val Val Leu Val Pro Asp Gly
               20                  25                  30
   Leu Gly Glu Cys Gln Met Phe Asp Ser Ser Val Ser Gln Ile Ala Ala
           35                  40                  45
   Gln Gly Phe Arg Val Thr Thr Phe Asp Met Pro Gly Met Ser Arg Ser
       50                  55                  60
   Ala Lys Ala Pro Pro Glu Thr Tyr Thr Glu Val Thr Ala Gln Lys Leu
   65                  70                  75                  80
   Ala Ser Tyr Val Ile Ser Val Leu Asp Ala Leu Asp Ile Lys His Ala
                   85                  90                  95
   Thr Val Trp Gly Cys Ser Ser Gly Ala Ser Thr Val Val Ala Leu Leu
               100                 105                 110
   Leu Gly Tyr Pro Asp Arg Ile Arg Asn Ala Met Cys His Glu Leu Pro
           115                 120                 125
   Thr Lys Leu Leu Asp His Leu Ser Asn Thr Ala Val Leu Glu Asp Glu
       130                 135                 140
   Glu Ile Ser Lys Ile Leu Ala Asn Val Met Leu Asn Asp Val Ser Gly
   145                 150                 155                 160
   Gly Ser Glu Ala Trp Gln Ala Met Gly Asp Glu Val His Ala Arg Leu
                   165                 170                 175
   His Lys Asn Tyr Pro Val Trp Ala Arg Gly Tyr Pro Arg Thr Ile Pro
               180                 185                 190
   Pro Ser Ala Pro Val Lys Asp Leu Glu Ala Leu Arg Gly Lys Pro Leu
           195                 200                 205
   Asp Trp Thr Val Gly Ala Ala Thr Pro Thr Glu Ser Phe Phe Asp Asn
       210                 215                 220
   Ile Val Thr Ala Thr Lys Ala Gly Val Asn Ile Gly Leu Leu Pro Gly
   225                 230                 235                 240
   Met His Phe Pro Tyr Val Ser His Pro Asp Val Phe Ala Lys Tyr Val
                   245                 250                 255
   Val Glu Thr Thr Gln Lys His Leu
               260
<210> 5
   <211> 906
   <212> DNA
   <213> Artificial Sequence
<220>
   <223> nucleic acid sequence BTA-hydrolase 2
<400> 5
   atggctgtta tgaccccgcg tcgtgaacgt tcttctctgc tgtctcgtgc tctgcgtttc      60
   accgctgctg ctgctaccgc tctggttacc gctgtttctc tggctgctcc ggctcacgct     120
   gctaacccgt acgaacgtgg tccgaacccg accgacgctc tgctggaagc tcgttctggt     180
   ccgttctctg tttctgaaga acgtgcttct cgtttcggtg ctgacggttt cggtggtggt     240
   accatctact acccgcgtga aaacaacacc tacggtgctg ttgctatctc tccgggttac     300
   accggtaccc aggcttctgt tgcttggctg ggtaaacgta tcgcttctca cggtttcgtt     360
   gttatcacca tcgacaccaa caccaccctg gaccagccgg actctcgtgc tcgtcagctg     420
   aacgctgctc tggactacat gatcaacgac gcttcttctg ctgttcgttc tcgtatcgac     480
   tcttctcgtc tggctgttat gggtcactct atgggtggtg gtggttctct gcgtctggct     540
   tctcagcgtc cggacctgaa agctgctatc ccgctgaccc cgtggcacct gaacaaaaac     600
   tggtcttctg ttcgtgttcc gaccctgatc atcggtgctg acctggacac catcgctccg     660
   gttctgaccc acgctcgtcc gttctacaac tctctgccga cctctatctc taaagctcac     720
   ctggaactgg acggtgctac ccacttcgct ccgaacatcc cgaacaaaat catcggtaaa     780
   tactctgttg cttggctgaa acgtttcgtt gacaacgaca cccgttacac ccagttcctg     840
   tgcccaggtc cgcgtgacgg tctgttcggt gaagttgaag aataccgttc tacctgcccg     900
   ttctga                                                                906
<210> 6
   <211> 301
   <212> PRT
   <213> Artificial Sequence
<220>
   <223> amino acid sequence of BTA-hydrolase 2
<400> 6
   Met Ala Val Met Thr Pro Arg Arg Glu Arg Ser Ser Leu Leu Ser Arg
   1               5                   10                  15
   Ala Leu Arg Phe Thr Ala Ala Ala Ala Thr Ala Leu Val Thr Ala Val
               20                  25                  30
   Ser Leu Ala Ala Pro Ala His Ala Ala Asn Pro Tyr Glu Arg Gly Pro
           35                  40                  45
   Asn Pro Thr Asp Ala Leu Leu Glu Ala Arg Ser Gly Pro Phe Ser Val
       50                  55                  60
   Ser Glu Glu Arg Ala Ser Arg Phe Gly Ala Asp Gly Phe Gly Gly Gly
   65                  70                  75                  80
   Thr Ile Tyr Tyr Pro Arg Glu Asn Asn Thr Tyr Gly Ala Val Ala Ile
                   85                  90                  95
   Ser Pro Gly Tyr Thr Gly Thr Gln Ala Ser Val Ala Trp Leu Gly Lys
               100                 105                 110
   Arg Ile Ala Ser His Gly Phe Val Val Ile Thr Ile Asp Thr Asn Thr
           115                 120                 125
   Thr Leu Asp Gln Pro Asp Ser Arg Ala Arg Gln Leu Asn Ala Ala Leu
       130                 135                 140
   Asp Tyr Met Ile Asn Asp Ala Ser Ser Ala Val Arg Ser Arg Ile Asp
   145                 150                 155                 160
   Ser Ser Arg Leu Ala Val Met Gly His Ser Met Gly Gly Gly Gly Ser
                   165                 170                 175
   Leu Arg Leu Ala Ser Gln Arg Pro Asp Leu Lys Ala Ala Ile Pro Leu
               180                 185                 190
   Thr Pro Trp His Leu Asn Lys Asn Trp Ser Ser Val Arg Val Pro Thr
           195                 200                 205
   Leu Ile Ile Gly Ala Asp Leu Asp Thr Ile Ala Pro Val Leu Thr His
       210                 215                 220
   Ala Arg Pro Phe Tyr Asn Ser Leu Pro Thr Ser Ile Ser Lys Ala His
   225                 230                 235                 240
   Leu Glu Leu Asp Gly Ala Thr His Phe Ala Pro Asn Ile Pro Asn Lys
                   245                 250                 255
   Ile Ile Gly Lys Tyr Ser Val Ala Trp Leu Lys Arg Phe Val Asp Asn
               260                 265                 270
   Asp Thr Arg Tyr Thr Gln Phe Leu Cys Pro Gly Pro Arg Asp Gly Leu
   275 280 285
   Phe Gly Glu Val Glu Glu Tyr Arg Ser Thr Cys Pro Phe
   290 295 300
<210> 7
   <211> 912
   <212> DNA
   <213> Artificial Sequence
<220>
   <223> nucleic acid sequence lipase
<400> 7
   atgcactcta ccccgcgtac cggtaccacc caggctccgg gtctgtctcg tcgtctggct 60
   gcttctgctg ctgctgttgc tgctgttgtt ggtctgacca ccctgtctac ccctggtgct 120
   caggctgcta acccgtacga acgtggtccg gctccgacca acgcttctat cgaggcttct 180
   cgtggtccgt acgctacctc tcagacctct gtttcttctc tggttgcttc tggtttcggt 240
   ggtggtacca tctactaccc gacctctacc gctgacggta ccttcggtgc tgttgttatc 300
   tctccgggtt tcaccgctta ccagtcttct atcgcttggc tgggtccgcg tctggcttct 360
   cagggtttcg ttgttttcac catcgacacc aacaccaccc tggaccagcc ggactctcgt 420
   ggtcgtcagc tgctgtctgc tctggactac ctgacccagc gttcttctgt tcgtacccgt 480
   gttgacgcta cccgtctggg tgttatgggt cactctatgg gtggtggtgg ttctctggaa 540
   gctgctaaat ctcgtacctc tctgaaagct gctatcccgc tgaccggttg gaacaccgac 600
   aaaacctggc cggaactgcg taccccgacc ctggttgttg gtgctgacgg tgacaccgtt 660
   gctccggttg ctacccactc taaaccgttc tacgaatctc tgccgggttc tctggacaag 720
   gcttacctgg aactgcgtgg tgcttctcac ttcaccccga acacctctga caccaccatc 780
   gctaaatact ctatctcttg gctgaaacgt ttcatcgact ctgacacccg ttacgaacag 840
   ttcctgtgcc cgatcccgcg tccgtctctg accatcgctg aataccgtgg tacctgcccg 900
   cacacctctt ga 912
<210> 8
   <211> 303
   <212> PRT
   <213> Artificial Sequence
<220>
   <223> the amino acid sequence of lipase
<400> 8
   Met His Ser Thr Pro Arg Thr Gly Thr Thr Gln Ala Pro Gly Leu Ser
   1               5                   10                  15
   Arg Arg Leu Ala Ala Ser Ala Ala Ala Val Ala Ala Val Val Gly Leu
               20                  25                  30
   Thr Thr Leu Ser Thr Pro Gly Ala Gln Ala Ala Asn Pro Tyr Glu Arg
           35                  40                  45
   Gly Pro Ala Pro Thr Asn Ala Ser Ile Glu Ala Ser Arg Gly Pro Tyr
       50                  55                  60
   Ala Thr Ser Gln Thr Ser Val Ser Ser Leu Val Ala Ser Gly Phe Gly
   65                  70                  75                  80
   Gly Gly Thr Ile Tyr Tyr Pro Thr Ser Thr Ala Asp Gly Thr Phe Gly
                   85                  90                  95
   Ala Val Val Ile Ser Pro Gly Phe Thr Ala Tyr Gln Ser Ser Ile Ala
               100                 105                 110
   Trp Leu Gly Pro Arg Leu Ala Ser Gln Gly Phe Val Val Phe Thr Ile
           115                 120                 125
   Asp Thr Asn Thr Thr Leu Asp Gln Pro Asp Ser Arg Gly Arg Gln Leu
       130                 135                 140
   Leu Ser Ala Leu Asp Tyr Leu Thr Gln Arg Ser Ser Val Arg Thr Arg
   145                 150                 155                 160
   Val Asp Ala Thr Arg Leu Gly Val Met Gly His Ser Met Gly Gly Gly
                   165                 170                 175
   Gly Ser Leu Glu Ala Ala Lys Ser Arg Thr Ser Leu Lys Ala Ala Ile
               180                 185                 190
   Pro Leu Thr Gly Trp Asn Thr Asp Lys Thr Trp Pro Glu Leu Arg Thr
           195                 200                 205
   Pro Thr Leu Val Val Gly Ala Asp Gly Asp Thr Val Ala Pro Val Ala
       210                 215                 220
   Thr His Ser Lys Pro Phe Tyr Glu Ser Leu Pro Gly Ser Leu Asp Lys
   225                 230                 235                 240
   Ala Tyr Leu Glu Leu Arg Gly Ala Ser His Phe Thr Pro Asn Thr Ser
                   245                 250                 255
   Asp Thr Thr Ile Ala Lys Tyr Ser Ile Ser Trp Leu Lys Arg Phe Ile
   260 265 270
   Asp Ser Asp Thr Arg Tyr Glu Gln Phe Leu Cys Pro Ile Pro Arg Pro
   275 280 285
   Ser Leu Thr Ile Ala Glu Tyr Arg Gly Thr Cys Pro His Thr Ser
   290 295 300
<210> 9
   <211> 915
   <212> DNA
   <213> Artificial Sequence
<220>
   <223> nucleic acid sequence PBS A depolymerase
<400> 9
   atgcacctgc cgcgttctcg ttgggacatc ccgttcaaag aagaaaccac catgacccac 60
   cacttctctg ttcgtgctct gctggctgct ggtgctctgc tggcttctgc tgctgtttct 120
   gctcagacca acccgtacga acgtggtccg gctccgacca cctcttctct ggaagcatct 180
   cgtggtccgt tctcttacca gtctttcacc gtttctcgtc cgtctggtta ccgtgctggt 240
   accgtttact acccgaccaa cgctggtggt ccggttggtg ctatcgctat cgttccgggt 300
   ttcaccgctc gtcagtcttc tatcaactgg tggggtccgc gtctggcttc tcacggtttc 360
   gttgttatca ccatcgacac caactctacc ctggaccagc cggactctcg ttctcgtcag 420
   cagatggctg ctctgtctca ggttgctacc ctgtctcgta cctcttcttc tccgatctac 480
   aacaaagttg acacctctcg tctgggtgtt atgggttggt ctatgggtgg tggtggttct 540
   ctgatctctg ctcgtaacaa cccgtctatc aaagctgctg ctccgcaggc tccgtggtct 600
   gcttctaaaa acttctcttc tctgaccgtt ccgaccctga tcatcgcttg cgaaaacgac 660
   accatcgctc cggttaacca gcacgctgac accttctacg actctatgtc tcgtaacccg 720
   cgtgagttcc tggaaatcaa caacggttct cactcttgcg ctaactctgg taactctaac 780
   caggctctgc tgggtaaaaa aggtgttgct tggatgaaac gtttcatgga caacgaccgt 840
   cgttacacct ctttcgcttg ctctaacccg aactcttaca acgtttctga cttccgtgtt 900
   gctgcttgca actga 915
<210> 10
   <211> 304
   <212> PRT
   <213> Artificial Sequence
<220>
   <223> amino acid sequence of PBS A depolymerase
<400> 10
   Met His Leu Pro Arg Ser Arg Trp Asp Ile Pro Phe Lys Glu Glu Thr
   1               5                   10                  15
   Thr Met Thr His His Phe Ser Val Arg Ala Leu Leu Ala Ala Gly Ala
               20                  25                  30
   Leu Leu Ala Ser Ala Ala Val Ser Ala Gln Thr Asn Pro Tyr Glu Arg
           35                  40                  45
   Gly Pro Ala Pro Thr Thr Ser Ser Leu Glu Ala Ser Arg Gly Pro Phe
       50                  55                  60
   Ser Tyr Gln Ser Phe Thr Val Ser Arg Pro Ser Gly Tyr Arg Ala Gly
   65                  70                  75                  80
   Thr Val Tyr Tyr Pro Thr Asn Ala Gly Gly Pro Val Gly Ala Ile Ala
                   85                  90                  95
   Ile Val Pro Gly Phe Thr Ala Arg Gln Ser Ser Ile Asn Trp Trp Gly
               100                 105                 110
   Pro Arg Leu Ala Ser His Gly Phe Val Val Ile Thr Ile Asp Thr Asn
           115                 120                 125
   Ser Thr Leu Asp Gln Pro Asp Ser Arg Ser Arg Gln Gln Met Ala Ala
       130                 135                 140
   Leu Ser Gln Val Ala Thr Leu Ser Arg Thr Ser Ser Ser Pro Ile Tyr
   145                 150                 155                 160
   Asn Lys Val Asp Thr Ser Arg Leu Gly Val Met Gly Trp Ser Met Gly
                   165                 170                 175
   Gly Gly Gly Ser Leu Ile Ser Ala Arg Asn Asn Pro Ser Ile Lys Ala
               180                 185                 190
   Ala Ala Pro Gln Ala Pro Trp Ser Ala Ser Lys Asn Phe Ser Ser Leu
           195                 200                 205
   Thr Val Pro Thr Leu Ile Ile Ala Cys Glu Asn Asp Thr Ile Ala Pro
       210                 215                 220
   Val Asn Gln His Ala Asp Thr Phe Tyr Asp Ser Met Ser Arg Asn Pro
   225                 230                 235                 240
   Arg Glu Phe Leu Glu Ile Asn Asn Gly Ser His Ser Cys Ala Asn Ser
                   245                 250                 255
   Gly Asn Ser Asn Gln Ala Leu Leu Gly Lys Lys Gly Val Ala Trp Met
   260 265 270
   Lys Arg Phe Met Asp Asn Asp Arg Arg Tyr Thr Ser Phe Ala Cys Ser
   275 280 285
   Asn Pro Asn Ser Tyr Asn Val Ser Asp Phe Arg Val Ala Ala Cys Asn
   290 295 300
<210> 11
   <211> 906
   <212> DNA
   <213> Artificial Sequence
<220>
   <223> nucleic acid sequence BTA-hydrolase 1
<400> 11
   atggctgtga tgaccccccg ccgggagcgc tcttccctgc tctcccgagc tctgcaagtg      60
   acggctgcgg ctgccacagc gcttgtgacc gcggtcagcc tggccgcccc cgctcatgcc     120
   gccaacccct acgagcgcgg ccccaacccg accgacgccc tgctcgaagc cagcagcggc     180
   cccttctccg tcagcgagga gaacgtctcc cggttgagcg ccagcggctt cggcggcggc     240
   accatctact acccgcggga gaacaacacc tacggtgcgg tggcgatctc ccccggctac     300
   accggcactg aggcttccat cgcctggctg ggcgagcgca tcgcctccca cggcttcgtc     360
   gtcatcacca tcgacaccat caccaccctc gaccagccgg acagccgggc agagcagctc     420
   aacgccgcgc tgaaccacat gatcaaccgg gcgtcctcca cggtgcgcag ccggatcgac     480
   agcagccgac tggcggtcat gggccactcc atgggcggcg gcggcaccct gcgtctggcc     540
   tcccagcgtc ccgacctgaa ggccgccatc ccgctcaccc cgtggcacct caacaagaac     600
   tggagcagcg tcaccgtgcc gacgctgatc atcggggccg acctcgacac aatcgcgccg     660
   gtcgccacgc acgcgaaacc gttctacaac agcctgccga gctccatcag caaggcctac     720
   ctggagctgg acggcgcaac ccacttcgcc ccgaacatcc ccaacaagat catcggcaag     780
   tacagcgtcg cctggctcaa gcggttcgtc gacaacgaca cccgctacac ccagttcctc     840
   tgccccggac cgcgcgacgg actcttcggc gaggtcgaag agtaccgctc cacctgcccg     900
   ttctag                                                                906
<210> 12
   <211> 301
   <212> PRT
   <213> Artificial Sequence
<220>
   <223> amino acid sequence of BTA-hydrolase 1
<400> 12
   Met Ala Val Met Thr Pro Arg Arg Glu Arg Ser Ser Leu Leu Ser Arg
   1               5                   10                  15
   Ala Leu Gln Val Thr Ala Ala Ala Ala Thr Ala Leu Val Thr Ala Val
               20                  25                  30
   Ser Leu Ala Ala Pro Ala His Ala Ala Asn Pro Tyr Glu Arg Gly Pro
           35                  40                  45
   Asn Pro Thr Asp Ala Leu Leu Glu Ala Ser Ser Gly Pro Phe Ser Val
       50                  55                  60
   Ser Glu Glu Asn Val Ser Arg Leu Ser Ala Ser Gly Phe Gly Gly Gly
   65                  70                  75                  80
   Thr Ile Tyr Tyr Pro Arg Glu Asn Asn Thr Tyr Gly Ala Val Ala Ile
                   85                  90                  95
   Ser Pro Gly Tyr Thr Gly Thr Glu Ala Ser Ile Ala Trp Leu Gly Glu
               100                 105                 110
   Arg Ile Ala Ser His Gly Phe Val Val Ile Thr Ile Asp Thr Ile Thr
           115                 120                 125
   Thr Leu Asp Gln Pro Asp Ser Arg Ala Glu Gln Leu Asn Ala Ala Leu
       130                 135                 140
   Asn His Met Ile Asn Arg Ala Ser Ser Thr Val Arg Ser Arg Ile Asp
   145                 150                 155                 160
   Ser Ser Arg Leu Ala Val Met Gly His Ser Met Gly Gly Gly Gly Thr
                   165                 170                 175
   Leu Arg Leu Ala Ser Gln Arg Pro Asp Leu Lys Ala Ala Ile Pro Leu
               180                 185                 190
   Thr Pro Trp His Leu Asn Lys Asn Trp Ser Ser Val Thr Val Pro Thr
           195                 200                 205
   Leu Ile Ile Gly Ala Asp Leu Asp Thr Ile Ala Pro Val Ala Thr His
       210                 215                 220
   Ala Lys Pro Phe Tyr Asn Ser Leu Pro Ser Ser Ile Ser Lys Ala Tyr
   225                 230                 235                 240
   Leu Glu Leu Asp Gly Ala Thr His Phe Ala Pro Asn Ile Pro Asn Lys
                   245                 250                 255
   Ile Ile Gly Lys Tyr Ser Val Ala Trp Leu Lys Arg Phe Val Asp Asn
               260                 265                 270
   Asp Thr Arg Tyr Thr Gln Phe Leu Cys Pro Gly Pro Arg Asp Gly Leu
           275                 280                 285
   Phe Gly Glu Val Glu Glu Tyr Arg Ser Thr Cys Pro Phe
       290                 295                 300
<210> 13
   <211> 264
   <212> PRT
   <213> Artificial Sequence
<220>
   <223> amino acid sequence of Myco H03
<400> 13
   Ala Ala Asn Pro Tyr Glu Arg Gly Pro Ala Pro Thr Asn Ala Ser Ile
   1               5                   10                  15
   Glu Ala Ser Arg Gly Pro Tyr Ala Thr Ser Gln Thr Ser Val Ser Ser
               20                  25                  30
   Leu Val Ala Ser Gly Phe Gly Gly Gly Thr Ile Tyr Tyr Pro Thr Ser
           35                  40                  45
   Thr Ala Asp Gly Thr Phe Gly Ala Val Ala Ile Ser Pro Gly Tyr Thr
       50                  55                  60
   Gly Thr Gln Ala Ser Val Ala Trp Leu Gly Lys Arg Ile Ala Ser His
   65                  70                  75                  80
   Gly Phe Val Val Ile Thr Ile Asp Thr Asn Thr Thr Leu Asp Gln Pro
                   85                  90                  95
   Asp Ser Arg Ala Arg Gln Leu Asn Ala Ala Leu Asp Tyr Met Ile Asn
               100                 105                 110
   Asp Ala Ser Ser Ala Val Arg Ser Arg Ile Asp Ser Ser Arg Leu Ala
           115                 120                 125
   Val Met Gly His Ser Met Gly Gly Gly Gly Ser Leu Arg Leu Ala Ser
       130                 135                 140
   Gln Arg Pro Asp Leu Lys Ala Ala Ile Pro Leu Thr Pro Trp His Leu
   145                 150                 155                 160
   Asn Lys Asn Trp Ser Ser Val Arg Val Pro Thr Leu Ile Ile Gly Ala
                   165                 170                 175
   Asp Leu Asp Thr Ile Ala Pro Val Leu Thr His Ala Arg Pro Phe Tyr
               180                 185                 190
   Asn Ser Leu Pro Thr Ser Ile Ser Lys Ala His Leu Glu Leu Asp Gly
           195                 200                 205
   Ala Thr His Phe Ala Pro Asn Ile Pro Asn Lys Ile Ile Gly Lys Tyr
       210                 215                 220
   Ser Val Ala Trp Leu Lys Arg Phe Val Asp Asn Asp Thr Arg Tyr Thr
   225                 230                 235                 240
   Gln Phe Leu Cys Pro Gly Pro Arg Asp Gly Leu Phe Gly Glu Val Glu
                   245                 250                 255
   Glu Tyr Arg Ser Thr Cys Pro Phe
               260
<210> 14
   <211> 262
   <212> PRT
   <213> Artificial Sequence
<220>
   <223> amino acid sequence of Myco H04
<400> 14
   Ala Ala Asn Pro Tyr Glu Arg Gly Pro Ala Pro Thr Asn Ala Ser Ile
   1               5                   10                  15
   Glu Ala Ser Arg Gly Pro Tyr Ala Thr Ser Gln Thr Ser Val Ser Ser
               20                  25                  30
   Leu Val Ala Ser Gly Phe Gly Gly Gly Thr Ile Tyr Tyr Pro Thr Ser
           35                  40                  45
   Thr Ala Asp Gly Thr Phe Gly Ala Val Val Ile Ser Pro Gly Phe Thr
       50                  55                  60
   Ala Tyr Gln Ser Ser Ile Ala Trp Leu Gly Pro Arg Leu Ala Ser Gln
   65                  70                  75                  80
   Gly Phe Val Val Phe Thr Ile Asp Thr Asn Thr Thr Leu Asp Gln Pro
                   85                  90                  95
   Asp Ser Arg Gly Arg Gln Leu Leu Ser Ala Leu Asp Tyr Leu Thr Gln
   100 105 110
   Arg Ser Ser Val Arg Thr Arg Val Asp Ala Thr Arg Leu Gly Val Met
   115 120 125
   Gly His Ser Met Gly Gly Gly Gly Ser Leu Arg Leu Ala Ser Gln Arg
   130 135 140
   Pro Asp Leu Lys Ala Ala Ile Pro Leu Thr Pro Trp His Leu Asn Lys
   145 150 155 160
   Asn Trp Ser Ser Val Arg Val Pro Thr Leu Ile Ile Gly Ala Asp Leu
   165 170 175
   Asp Thr Ile Ala Pro Val Leu Thr His Ala Arg Pro Phe Tyr Asn Ser
   180 185 190
   Leu Pro Thr Ser Ile Ser Lys Ala His Leu Glu Leu Asp Gly Ala Thr
   195 200 205
   His Phe Ala Pro Asn Ile Pro Asn Lys Ile Ile Gly Lys Tyr Ser Val
   210 215 220
   Ala Trp Leu Lys Arg Phe Val Asp Asn Asp Thr Arg Tyr Thr Gln Phe
   225 230 235 240
   Leu Cys Pro Gly Pro Arg Asp Gly Leu Phe Gly Glu Val Glu Glu Tyr
   245 250 255
   Arg Ser Thr Cys Pro Phe
   260
<210> 15
   <211> 262
   <212> PRT
   <213> Artificial Sequence
<220>
   <223> the amino acid sequence of Myco H10
<400> 15
   Ala Ala Asn Pro Tyr Glu Arg Gly Pro Ala Pro Thr Asn Ala Ser Ile
   1 5 10 15
   Glu Ala Ser Arg Gly Pro Tyr Ala Thr Ser Gln Thr Ser Val Ser Ser
   20 25 30
   Leu Val Ala Ser Gly Phe Gly Gly Gly Thr Ile Tyr Tyr Pro Thr Ser
           35                  40                  45
   Thr Ala Asp Gly Thr Phe Gly Ala Val Val Ile Ser Pro Gly Phe Thr
       50                  55                  60
   Ala Tyr Gln Ser Ser Ile Ala Trp Leu Gly Pro Arg Leu Ala Ser Gln
   65                  70                  75                  80
   Gly Phe Val Val Phe Thr Ile Asp Thr Asn Thr Thr Leu Asp Gln Pro
                   85                  90                  95
   Asp Ser Arg Gly Arg Gln Leu Leu Ser Ala Leu Asp Tyr Leu Thr Gln
               100                 105                 110
   Arg Ser Ser Val Arg Thr Arg Val Asp Ala Thr Arg Leu Gly Val Met
           115                 120                 125
   Gly His Ser Met Gly Gly Gly Gly Ser Leu Glu Ala Ala Lys Ser Arg
       130                 135                 140
   Thr Ser Leu Lys Ala Ala Ile Pro Leu Thr Gly Trp Asn Thr Asp Lys
   145                 150                 155                 160
   Thr Trp Pro Glu Leu Arg Thr Pro Thr Leu Val Val Gly Ala Asp Gly
                   165                 170                 175
   Asp Thr Val Ala Pro Val Ala Thr His Ser Lys Pro Phe Tyr Glu Ser
               180                 185                 190
   Leu Pro Gly Ser Leu Asp Lys Ala Tyr Leu Glu Leu Arg Gly Ala Ser
           195                 200                 205
   His Phe Thr Pro Asn Thr Ser Asp Thr Thr Ile Ala Lys Tyr Ser Ile
       210                 215                 220
   Ser Trp Leu Lys Arg Phe Ile Asp Ser Asp Thr Arg Tyr Glu Gln Phe
   225                 230                 235                 240
   Leu Cys Pro Gly Pro Arg Asp Gly Leu Phe Gly Glu Val Glu Glu Tyr
                   245                 250                 255
   Arg Ser Thr Cys Pro Phe
               260
<210> 16
   <211> 263
   <212> PRT
   <213> Artificial Sequence
<220>
   <223> amino acid sequence of Myco H11
<400> 16
   Ala Gln Thr Asn Pro Tyr Glu Arg Gly Pro Ala Pro Thr Thr Ser Ser
   1               5                   10                  15
   Leu Glu Ala Ser Arg Gly Pro Phe Ser Val Ser Glu Glu Arg Thr Ser
               20                  25                  30
   Arg Phe Gly Ala Asp Gly Phe Gly Gly Gly Thr Ile Tyr Tyr Pro Arg
           35                  40                  45
   Glu Asn Asn Thr Tyr Gly Ala Val Ala Ile Ser Pro Gly Tyr Thr Gly
       50                  55                  60
   Thr Gln Ala Ser Val Ala Trp Leu Gly Lys Arg Ile Ala Ser His Gly
   65                  70                  75                  80
   Phe Val Val Ile Thr Ile Asp Thr Asn Thr Thr Leu Asp Gln Pro Asp
                   85                  90                  95
   Ser Arg Ala Arg Gln Leu Asn Ala Ala Leu Asp Tyr Met Ile Asn Asp
               100                 105                 110
   Ala Ser Ser Ala Val Arg Ser Arg Ile Asp Ser Ser Arg Leu Ala Val
           115                 120                 125
   Met Gly His Ser Met Gly Gly Gly Gly Ser Leu Arg Leu Ala Ser Gln
       130                 135                 140
   Arg Pro Asp Leu Lys Ala Ala Ile Pro Leu Thr Pro Trp His Leu Asn
   145                 150                 155                 160
   Lys Asn Trp Ser Ser Val Arg Val Pro Thr Leu Ile Ile Gly Ala Asp
                   165                 170                 175
   Leu Asp Thr Ile Ala Pro Val Leu Thr His Ala Arg Pro Phe Tyr Asn
               180                 185                 190
   Ser Leu Pro Thr Ser Ile Ser Lys Ala His Leu Glu Leu Asp Gly Ala
           195                 200                 205
   Thr His Phe Ala Pro Asn Ile Pro Asn Lys Ile Ile Gly Lys Tyr Ser
       210                 215                 220
   Val Ala Trp Leu Lys Arg Phe Val Asp Asn Asp Thr Arg Tyr Thr Gln
   225                 230                 235                 240
   Phe Leu Cys Pro Gly Pro Arg Asp Gly Leu Phe Gly Glu Val Gly Glu
                   245                 250                 255
   Tyr Arg Ser Thr Cys Pro Phe
               260

## Description

### FIELD OF THE INVENTION

The present invention relates to ZON lactonase enzymes (ZONASES) for degrading mycotoxins.

### BACKGROUND OF THE INVENTION

Several plant pathogenic and/or post-harvest Fusarium species on cereals produce toxic substances of considerable concern to livestock and poultry producers, e.g., deoxynivalenol, T-2 toxin, HT-2 toxin, diacetoxyscirpenol and zearalenone (ZON). Zearalenone is found worldwide in a number of cereal crops, such as maize, barley, oats, wheat, rye, rice, millet and sorghum. Zearalenone production does not seem to occur in significant amounts prior to harvest, but under proper environmental conditions, it is readily produced on corn and small grains in storage.

Mycotoxin formation may occur when the causative fungi grow on crops in the field, at harvest, in storage, or during feed processing; essentially whenever favorable conditions for their formation prevail. Generalizations about geographical distribution of particular types of mycotoxins are difficult due to widespread distribution of the causative fungi. However, aflatoxins and fumonisin tend to prevail in warmer climates, while cooler regions with higher moisture are subject to ochratoxin, zearalenone, vomitoxin (deoxynivalenol, DON), T2 toxin, and others. Each mycotoxin has its own particular effect, and all can be devastating. Co-contamination by one or more types of mycotoxin occurs naturally, and exerts a greater negative impact on health and productivity of livestock than contamination by individual mycotoxins.

Zearalenone is heat-stable, and it is not destroyed by long storage, roasting, or by the addition of propionic acid or mold retardants. Despite their structural dissimilarity to the steriodal estrogens, zearalenone and several of its derivatives possess estrogenic activity. Zearalenone undergoes a folding such that hydroxyl or potential hydroxyl groups become appropriately orientated to facilitate binding to tissue receptors that normally bind estrogens.

Zearalenone is the primary toxin causing infertility, abortion or other breeding problems, especially in swine. The symptoms are especially severe in prepubertal gilts including enlarged mammae, swelling of uterus and vulva, and atrophy of the ovaries. In severe cases, prolapse of the vulva and rectum may occur. Boars exhibit enlarged mammae and atrophied testes. The mycotoxin is present in the meat from animals feeding on contaminated grain as well as in bread baked from contaminated wheat. While cases of poisoning of humans are rare there is concern about the effect of the long term exposure of humans to such an estrogenic activity.

Microbial enzymes are a resource for many biotechnological applications.

Microbial strains or their enzymes may be used for degradative (bioremediation) or synthetic (biotransformation) purposes. Modern bioremediation or biotransformation strategies may even involve microbial catalysts or strains designed by protein engineering or pathway engineering. Prerequisite for developing such modern tools of biotechnology is a comprehensive understanding of microbial metabolic pathways, of the structure and function of enzymes, and of the molecular mechanisms of biocatalysis.

The *in vitro* inactivation of mycotoxins by cultivating the substrate with selected enzymes in a liquid, slurry or paste, using epoxidase, lactonase, laccase or cutinase is disclosed in WO9612414, WO2009077447 and WO2009080701. Takahashi-Ando et al. (Biochem. J. 2002; 364: 1 -6) describe a lactonohydrolase for the detoxification of zearalenone at a pH optimum of pH 9-10. At low pH (< pH 4.5) the enzyme was unstable and irreversibly inactivated.

A method of removing mycotoxins from animal feed is described in WO99053772 whereby a modified yeast cell walls extract and a mineral clay is added to the feed product.

A feed additive based on dried yeast and sea algae is provided by Biomin GmbH (Herzogenburg, Austria) for deactivating mycotoxins.

Takahashi-Ando et al. (Appl Environ Microbiol. 2004 Jun;70(6):3239-45) discloses the detoxification with recombinant E.coli and transgenic rice cells at pH 6.2.

An esterase is disclosed in WO0123581 which is similar to the triacylglycerol hydrolase.

There is still a need for further means of detoxifying animal food products. It is thus the objective of the invention to provide for improved enzymatic detoxification of mycotoxins in feed products.

### SUMMARY OF THE INVENTION

The inventors of the present invention have discovered that ZON in a food product can be degraded by treating the food product with zonase from the serine hydrolase superfamily that is surprisingly GI stable to enable *in situ* mycotoxin deactivation in or upon passage of the gastrointestinal tract.

Accordingly, in a first aspect the invention provides for a food supplement for treating a ZON contaminated feed product comprising an enzyme product containing a zonase according to any one of claims 1 to 6.

The feed supplement according to the invention preferably contains the enzyme in the purified form, with a purity of at least 70%, preferably of at least 80% and most preferred of at least 90%.

The feed supplement according to the invention preferably contains a BTA-1 hydrolase or a BTA-2 hydrolase.

Specifically the enzyme as used according to the invention has a pH stability in the range of pH 3 to 8 and a protease stability in the presence of pancreatic enzymes, e.g. is GI stable. Thus, the enzyme is stable under conditions comparable to passage of the stomach (acidic) and small intestine (neutral). The enzyme advantageously is stable against pepsin and pancreatin digestion, essentially maintaining its ZON detoxification activity. The enzyme advantageously is active at low pH, preferably in the range of pH 3 to 7.

The enzyme preferably has ZON detoxification activity as measured in a cell based assay, specifically using a standard proliferation assay to detect the decrease of ZON estrogenic activity with the human breast adenocarcinoma cell line MCF-7.

A further aspect of the invention is the use of a zonase for in vitro or in situ detoxification of a mycotoxin. This object is achieved with the use of a recombinant zonase having pH stability in the range of pH 3 to 8 and a protease stability in the presence of pancreatic enzymes for treating food products wherein the zonase is any enzyme which is capable of hydrolysing the lactone ring of Zearalenone (ZON) and is selected from the group consisting of hydrolases, lipases, esterases and depolymerases and
wherein the zonase is pH stable if it retains 40 % of residua! activity after incubation for 30 min at pH 3 and at 37 °C; and
wherein the protease stability is achieved when the zonase retains either at least 84 % residual activity after incubation of 12,5 µl 0,5 mg/ml purified zonase mixed with 12,5 µl cleared lysate of *E. coli* and 25 µl 1 mg/ml pepsin solution (pH 2) for 30 min at 37 °C or at least 27 % residua! activity after incubation of 12,5 µl purified zonase solution mixed with 12,5 µl cleared lysate of *E.coli* and 25 µl 5mg/ml pancreatin solution (pH 8) for 30 min at 37 °C,
wherein the zonase is an enzyme of the Moraxella lipase 1 like enzyme family.

A preferred enzyme of the Moraxella lipase 1 like enzyme family is selected from the group of BTA-hydrolase 1 from *Thermobifida fusca,* BTA-hydrolase 2 form *Thermobifida fusca,* lipase form *Streptomyces exfoliates,* PBS A depolymerase from *Acidovoras delafieldii* and triacylglycerol hydrolase from *Streptomyces so*., or functionally active variants thereof.

In an aspect a variant of BTA-2 hydrolase is used with a sequence identity of at least 50%, preferably at least 60%, or at least 70%, or at least 80% or at least 90% to the parent BTA-2 hydrolase.

Therefore, a GI stable BTA-2 hydrolase variant with ZON detoxification activity is provided, having a sequence identity of at least 50%, preferably at least 60%, or at least 70%, or at least 80% or at least 90% compared to the parent BTA-2 hydrolase.

A further aspect of the invention is a GI stable BTA-2 hydrolase variant with ZON detoxification activity according to the invention, having at least 25 consecutive amino acids selected from the amino acid sequence as set forth in SEQ ID NO: 6 and at least 25 consecutive amino acids selected from the amino acid sequence as set forth in SEQ ID NO: 10 or SEQ ID NO: 8.

A further aspect is a GI stable BTA-2 hydrolase variant with ZON detoxification activity according to the invention, having at least 50 consecutive amino acids selected from the amino acid sequence as set forth in SEQ ID NO: 6 and at least 25 consecutive amino acids selected from the amino acid sequence as set forth in SEQ ID NO: 10 or SEQ ID NO: 8.

Another aspect is a GI stable BTA-2 hydrolase variant with ZON detoxification activity according to the invention, having at least 25 consecutive amino acids selected from the amino acid sequence as set forth in SEQ ID NO: 6 and at least 50 consecutive amino acids selected from the amino acid sequence as set forth in SEQ ID NO: 10 or SEQ ID NO: 8.

Specifically preferred is a BTA-2 hydrolase variant selected from the group consisting of SEQ ID NO: 13, 14, 15 and 16.

There is further provided a food product comprising grain and at least one recombinant GI stable serine hydrolase enzyme, such as those used in the food supplement according to the invention.

The food product according to the invention preferably contains the enzyme in a dry mixture, e.g. as a premixed animal feed product or in a kit of parts containing (a) the grain product and (b) the feed supplement, which may be admixed to the grain product in the desired ratio. Preferably the grain is selected from corn, wheat, barley, rye, rice, sorghum and millet.

There is further provided a method of preparing a grain based food product, wherein the grain is treated with a recombinant GI stable serine hydrolase enzyme, such as used in the food supplement according to the invention. The food product may be treated *in vitro* or *in situ,* e.g. in the process of feeding and/or digesting, to effectively detoxify eventually present mycotoxins *in vivo.*

It is preferred that the enzyme is added to the grain in a dry mixture to obtain a premixed product commercialized as a ready-to-use mixture, or readily prepared before feeding.

Thus, according to the invention there is further provided a new use for a GI stable recombinant zonase for in situ detoxification of a mycotoxin upon feeding.

### BRIEF DESCRIPTION OF THE DRAWINGS

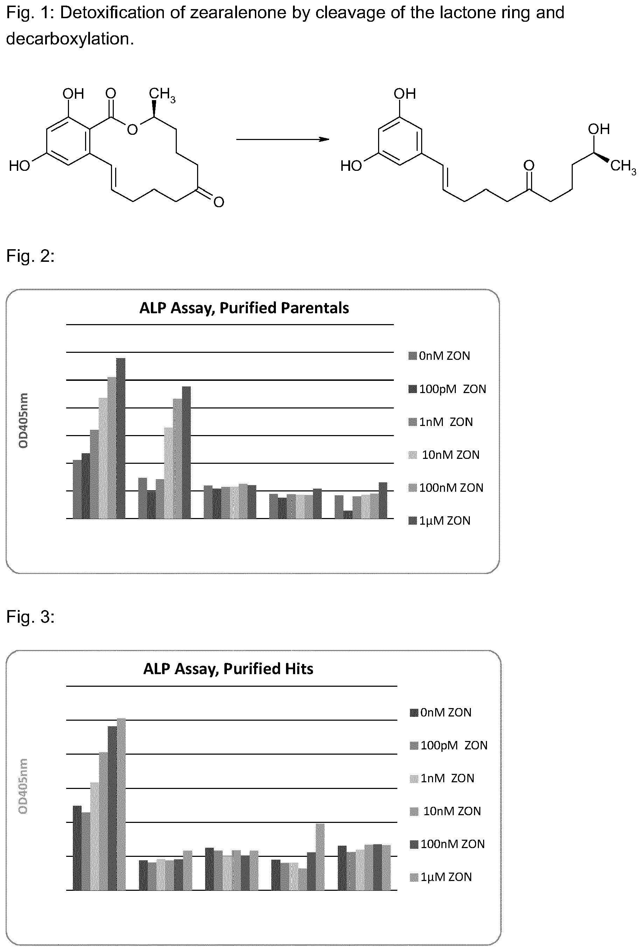

Figure 1 shows the detoxification of zearalenone by cleavage of the lactone ring and subsequent decarboxylation.
Figure 2 shows the zearalenone degrading activity of the parental enzymes.
Figure 3 shows the zearalenone degrading activity of the purified hits. The zearalenone degrading activity of the hits is comparable with the activity of the parental enzymes.

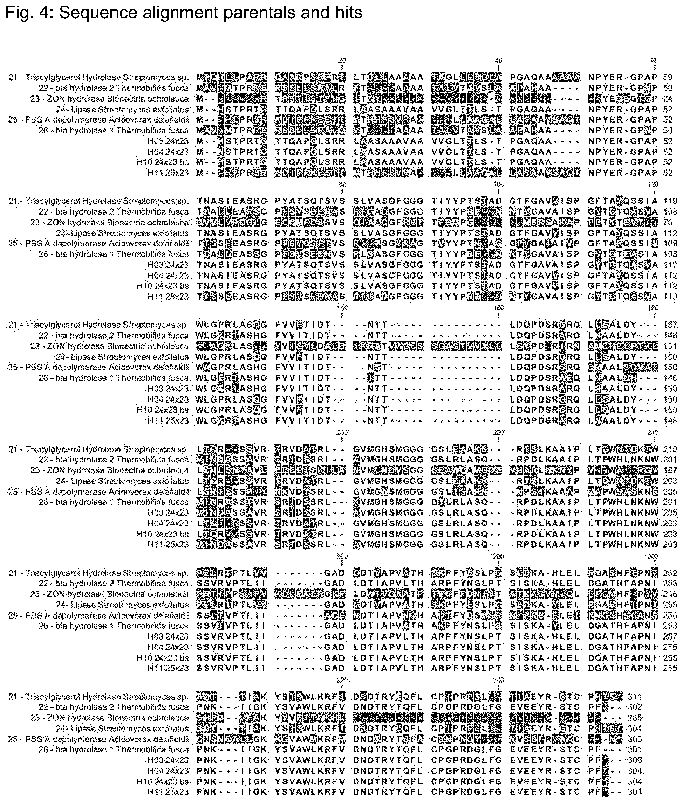

Figure 4 Sequence alignment of parentals and hits

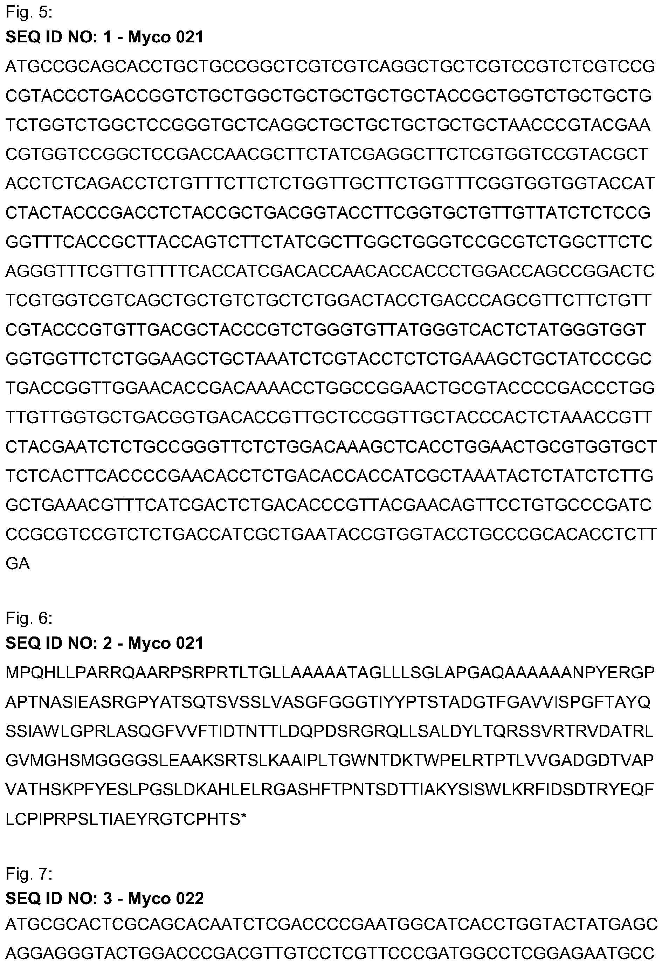

Figure 5 shows the nucleic acid sequence of triacylglycerol hydrolase (Myco 021)
Figure 6 shows the amino acid seqence of triacylglycerol hydrolase (Myco 021)
Figure 7 shows the nucleic acid sequence of zearalenone hydrolase (Myco 022)

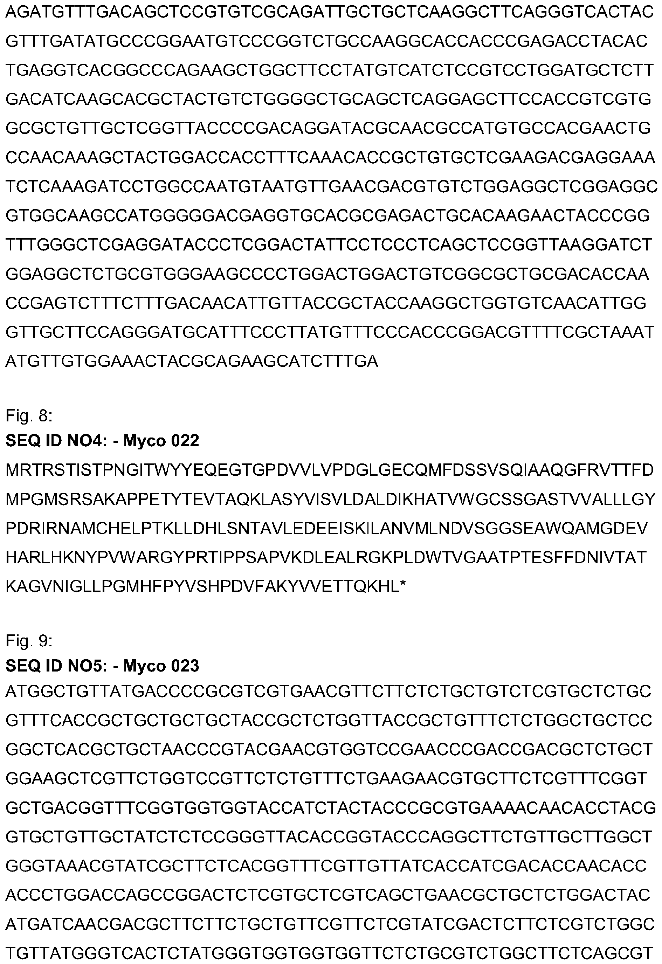

Figure 8 shows the amino acid sequence of zearalenone hydrolase (Myco 022)
Figure 9 shows the nucleic acid sequence BTA-hydrolase 2 (Myco 023)

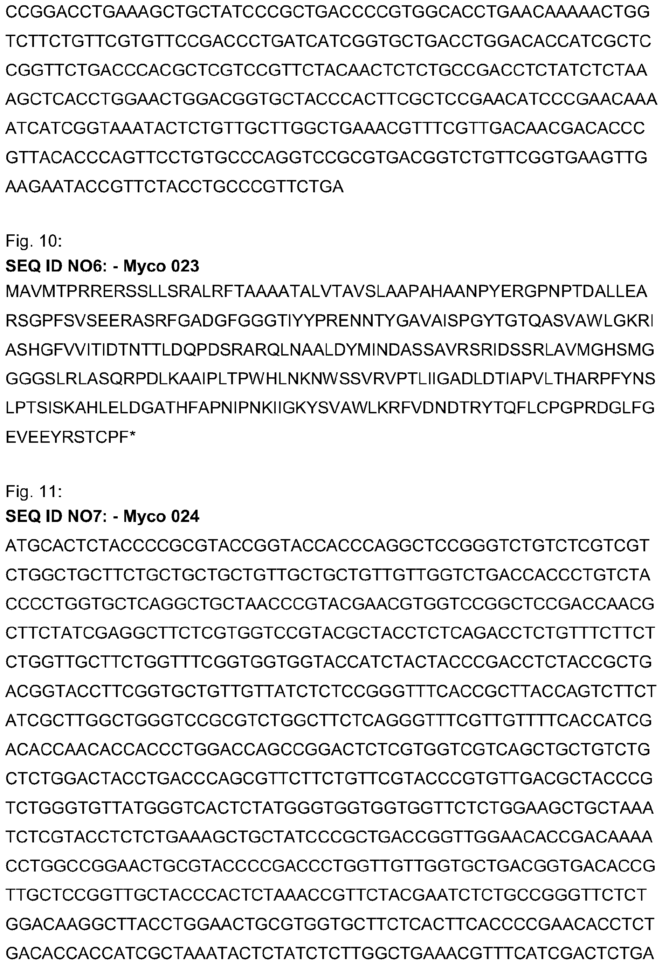

Figure 10 shows the amino acid sequence of BTA-hydrolase 2 (Myco 023)
Figure 11 shows the nucleic acid sequence lipase (Myco 024)

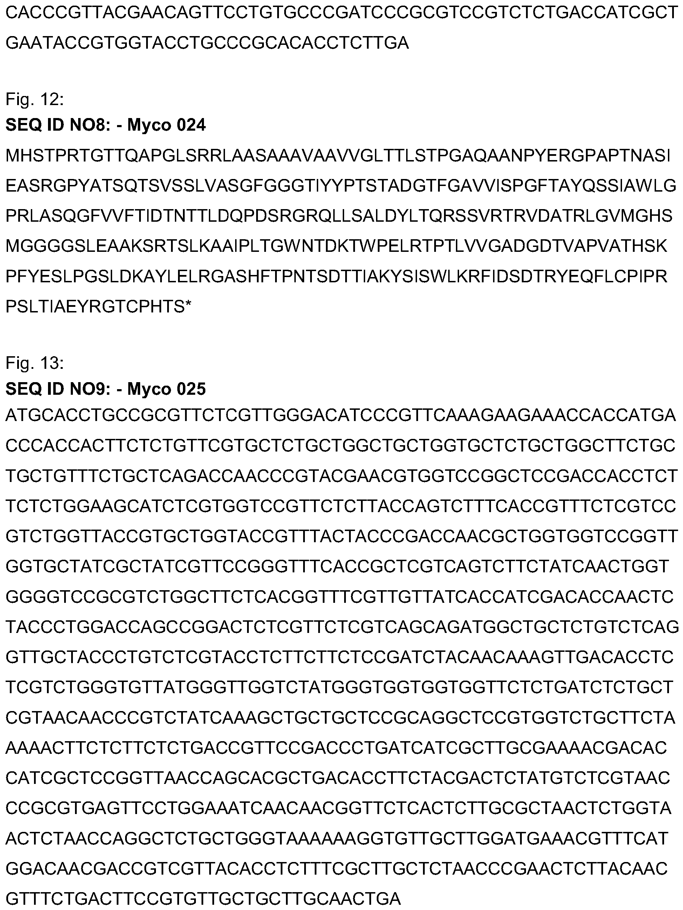

Figure 12 shows the amino acid sequence of lipase (Myco 024)
Figure 13 shows the nucleic acid sequence PBS A depolymerase (Myco 025)

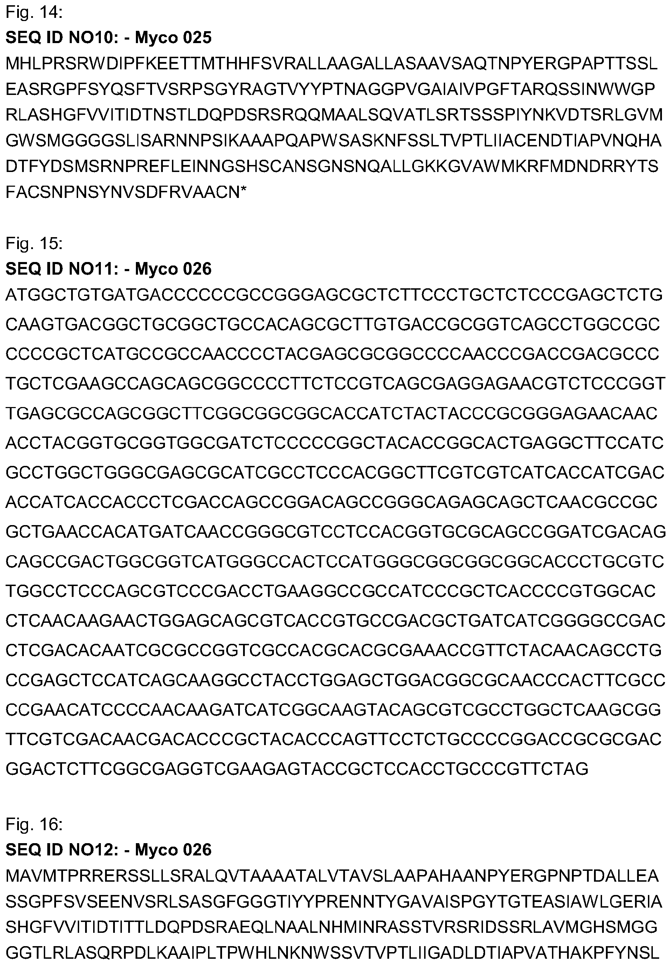

Figure 14 shows the amino acid sequence of PBS A depolymerase (Myco 025)
Figure 15 shows the nucleic acid sequence BTA-hydrolase 1 (Myco 026)
Figure 16 shows the amino acid sequence of BTA-hydrolase 1 (Myco 026)

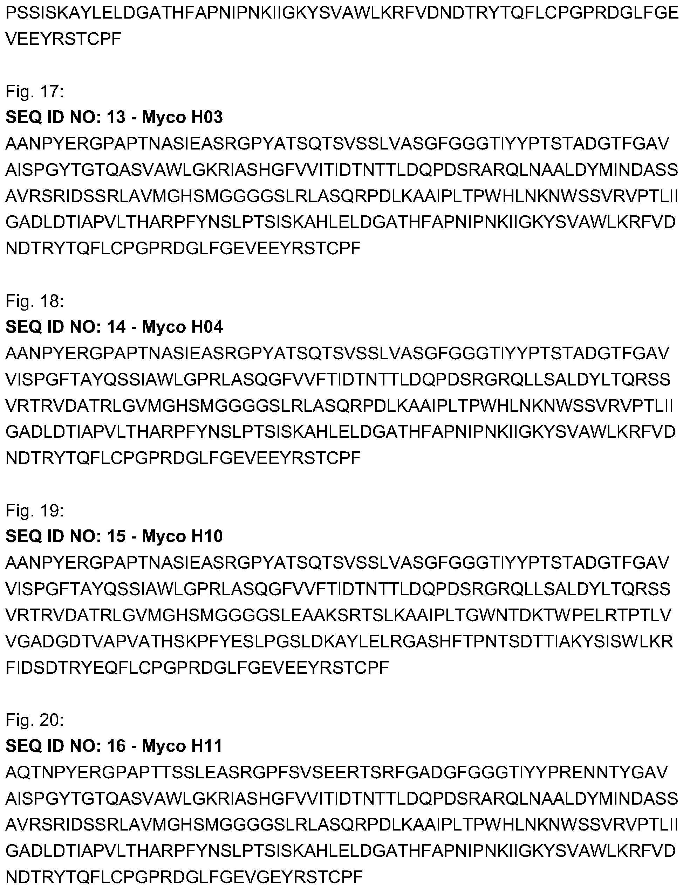

Figure 17 shows the amino acid sequence of Myco H03
Figure 18 shows the amino acid sequence of Myco H04
Figure 19 shows the amino acid sequence of Myco H10
Figure 20 shows the amino acid sequence of Myco H1 1

### DETAILED DESCRIPTION OF THE INVENTION

In the context of this invention the term "zearalenone" or ZON comprises the mycotoxin zearalenone produced from certain *Fusarium sp.* The IUPAC name is (4S, 72E)-15, 17-dihydroxy-4-methyl-3-oxabicyclo[12.4.0]octadeca-12, 15, 17, 19-tetraene- 2,8-dione. The term "zearalenone" also comprises any derivative of zearalenone which comprises an internal carboxylic ester bond susceptible for modification by a cutinase or a hydrolase.

The term "zonase" means any enzyme which is capable of hydrolyzing the lactone ring of zearalenone (ZON) or of its derivatives. Preferably, the zonase is a serine hydrolase superfamily enzyme.

The term "GI stable" means the enzyme is stable under conditions comparable to passage of the stomach (acidic) and small intestine (neutral). Specifically the enzyme as used according to the invention has a pH stability in the range of pH 3 to 8 and a protease stability in the presence of gastric and pancreatic enzymes. Thus, the enzyme advantageously is stable against pepsin and pancreatin digestion, essentially maintaining its ZON detoxification activity.

As used herein, "pH stability" refers to the ability of an enzyme to retain its activity at a particular pH. According to the invention, an enzyme is pH stable if it retains 40% of residual activity after incubation for 30 min at pH 3.

The term "animal" denotes all animals, including human beings. Examples of animals are cattle, (including but not limited to cows and calves); mono-gastric animals, e.g. pigs or swine (including, but not limited to, piglets, growing pigs, and sows); poultry such as turkeys and chicken (including but not limited to broiler chicks, layers); and fish (including but not limited to salmon).

The term "food" or "food product" means any feed, compound, preparation, mixture, or composition suitable for, or intended for intake by an animal. The food product may be a product which apart from an eventual unwanted level of ZON contamination is suitable for consumption by an animal. The food product can also be a product suspected of comprising an unwanted ZON level, and/or a product having an unknown ZON level, including products not comprising a detectable ZON level. Preferably the food product is a grain based product. Preferably the grain based product comprises cereal(s), e.g., one or more of corn, wheat, barley, rye, rice, sorghum and millet. Also preferred are grain based products comprising material derived from one or more of corn, wheat, barley, rye, rice, sorghum and millet. In one embodiment, the food product may e.g. be derived solely from cereal(s), and in another embodiment partly from legumes, e.g. from soybean, and partly from cereals. The grain based product may comprise whole or milled grain, e.g., wet or dry milled grain, including grain based product comprising fractions of wet or dry milled grain, e.g., gluten, protein, starch, and/or oil fractions. Also preferred are products comprising a by-product from brewing and/or fermentation processes, e.g., spent grain. Spent grain is the by-products from the production of alcoholic beverages and ethanol fuels. Brewers' spent grain (BSG) is the residue of beer making in breweries, which use malted barley as the major raw material. Distiller's spent grain (DSG) is the product left in distilleries after alcohol is removed by distillation from the fermented grains such as corn, wheat, barley, rice, and rye. Distiller's spent grain is also known as distiller's grain. Wet distiller's grain (WDG) is dried to produce dried distiller's grain (DDG) which is used primarily as animal feed. Under yet another embodiment of the present invention, the feed product is to be used to make processed feed based on starch or meal.

The term "food supplement" as used according to the invention refers to any nutritional or functional supplement to a food product that would improve the uptake or tolerability of the food. Food supplements as used herein typically provide for a functional food product that aids in the prevention and/or treatment of disease conditions associated with mycotoxins. Therefore, the food supplement according to the invention is also called a nutraceutical. In this regard the food supplement according to the invention is used as a nutraceutical to accomplish animal food and to reduce side effects resulting from an eventual mycotoxin contamination. The food supplement may be commercialized as an additive separate from the food product, with the advantage of the specific dosing to meet the animal's need on an individual or cohort basis. The food supplement may be admixed to the food product in a liquid, slurry (e.g. with water) or paste, and fed optionally after an incubation time of one to several hours. In a preferred embodiment, an aqueous solution or suspension is kept at a temperature that allows the enzyme to dissolve. The incubation time for *in vitro* processing preferably ranges from 1 minute to 1 week depending on the processing temperature. In many cases a treatment time in the range of 6 to 48 hours will be suitable. Preferably a reaction temperature is applied which is close to the optimum temperature of the enzyme employed. In numerous embodiments of the invention, temperatures in the range of 10 - 65°C, more preferably 25 - 50°C should be employed.

However, the food supplement may also be provided together with the food product as a dry mixture, either in a ready-to-use mixture or as a kit of parts, which provides for dry mixing onsite before feeding. To provide the mixture the food supplement is typically placed with the grain in a mixing device, such as a rotating drum, an electric mixer or a tumbler.

The ratio of admixing the food additive ranges from 0.01 g/t to 10 kg/t feed, preferred from 0.01 mg/t to 1000 g/t, more preferred from 0.1 mg/t to 10 g/t, most preferred from 1 mg/t to 1 g/t.

Other ingredients which, though not critical to the function of the enzyme, may be deemed as helpful in assisting in its roles of mycotoxin degradation and management which may be added to the composition include, but are not limited to, varying amounts of plant or algae extracts or diatomaceous earth.

Besides, the animal food additives of the invention preferably contain fat-soluble and/or water soluble vitamins, trace minerals, macro minerals, aroma compounds, stabilizers and co-substrates.

In a preferred embodiment the food supplement is provided in the dry form as a storage stable powder or granules. Preferably the dry enzyme preparation has a water content of less than 5%, more preferred less than 4%, more preferred less than 3%, even more preferred less than 2%.

The term "isolated enzyme" or "purified enzyme" as used herein refers to a polypeptide which is at least 30% pure, preferably at least 50% pure, more preferably at least 70% pure, even more preferably at least 80% pure, most preferably at least 90% pure, and even most preferably at least 95% pure, as determined by SDS-PAGE.

The food product according to the invention typically is provided in the food or feed grade quality. The grade quality is the quality characteristics of food or feed that is acceptable to animals. This includes external factors as appearance (size, shape, color, gloss, and consistency), texture and flavor. Quality standards also provide for an acceptable amount of contaminating substances. Besides ingredient quality, there are also sanitation requirements. It is important to ensure that the food processing environment is as clean as possible in order to produce the safest possible food for the consumer.

The zonase as used according to the invention optionally is of the serine hydrolase superfamily, which is one of the largest known enzyme families. This family particularly includes:
- serine proteases like trypsin, - lipases like pancreatic lipase, hormone sensitive lipase, and triacylglycerol lipase,
- cutinases,
- esterases,
- acetylcholinesterase,
- thioesterases,
- certain phospholipases like phospholipase A2, and
- some amidases like fatty acid amide hydrolase.

All of these enzymes share a catalytic mechanism that involves a catalytic triad consisting of a serine nucleophile that is activated by a proton relay involving an acidic residue (e.g. aspartate or glutamate) and a basic residue (usually histidine) although variations on this mechanism exist.

The present invention refers to any type of zonases of the serine hydrolase superfamily or functionally active variants thereof, which surprisingly have proven to be GI stable and exert a ZON degrading activity. As used herein the term "enzyme" always includes functionally active variants thereof.

For the purposes of the present invention the ZON degradation activity is determined in the unit of ZDA. One ZDA unit is defined as hydrolysis of 1 [mu][eta][iota][omicron][lota] ZON per hour at 37°C and pH 7. A suitable ZDA assay is a cell based standard proliferation assay, e.g. as exemplified below. ZDA activity may be determined in food and premix.

The enzyme as used according to the invention typically exerts a ZON degrading activity of at least 40 mU/mg enzyme.

GI stability may be determined by the pH stability and/or pepsin and/ or pancreatic protease stability, e.g. as exemplified below.

The estradiol-like effects of zearalenone are based on the lactone ring structure. ZON is converted to a non-estrogenic product by a detoxifying enzyme, which in- activates the molecule by enzymatic hydrolysis of the ring.

Cleavage of the lactone ring and subsequent decarboxylation could be accomplished by enzymes like lactone esterases or lactone decarboxylases. Some enzymes of the serine hydrolase superfamily active on zearalenone were now identified that were surprisingly GI stable.

In a first step enzyme candidates from various sources were selected, which are able to metabolize and detoxify zearalenone. Therefore a screening assay determining the biological activity of zearalenone as an estradiol-analogue was employed. Commercially available zearalenone (Sigma-Aldrich) was pre-incubated with enzymes of interest, and the mixture was incubated with cell lines susceptible to estrogen treatment (e. g. human breast cancer cell lines). The proliferation of the cells then served as read-out of the assay under high-throughput conditions. Potential hits were further characterized with regards to activity and stability and could be optimized by *in vivo* recombination to produce variants with improved features. These libraries of variants were individually screened for ZON reaction. Alternatively a set of variants may be screened, i.e. libraries of at least 20 zonases. Upon incubating the enzymes with the substrate zearalenone and screening for reaction products by HPLC, the desired detoxification product was verified by mass spectrometry (MS) analysis. The most preferred variants were then biologically characterized.

The esterase BTA-1 hydrolase (Myco 026; SEQ ID NO: 11, 12) of *Thermobifida fusca,* which is known to be a polyesterase, was found to effectively hydrolyze ZON. Other zonase-family members that share high homology in the active center and have some diversity over the whole sequence were identified by homology searches. Thereby, the lipase from *Streptomyces exfoliates* (Myco 24; SEQ ID NO: 7, 8), PBSA depolymerase from *Acidovorax delfieldii* (Myco 025 - SEQ ID NO: 9, 10) and BTA-2 hydrolase from *Thermobifida fusca* (Myco 023; SEQ ID NO: 5, 6) was found to be preferably used according to the invention. Further zonase with at least 50% sequence identity could be preferred, including functionally active variants to hydrolyze the mycotoxin.

Specifically the ZON detoxifying activity was detected for *Thermobifida fusca* BTA-hydrolase 1 (Myco 026; SEQ ID NO: 11, 12), an enzyme of the abH25.1 moxarella lipase 1 like family. Enzymatic cleavage of zearalenone and subsequent decarboxylation lead to a product representing a mass of approximately 290 g/mol. Other family members homologous to BTA-hydrolase 1 were identified by homology searches. It was found that all enzymes share high homology in the active centre, but have enough diversity over the whole sequence to ensure the generation of broad diversity by recombination.

As a preferred example lipase from Streptomyces exfoliatus and PBS A depolymerase from Acidovorax delafieldii were chosen for homologous recombination in addition to BTA hydrolase from Thermobifida fusca.

The lipase of *Streptomyces exfoliatus* lipase exhibits a typical canonical tertiary fold of an /[beta] hydrolase with a catalytic triad formed by the amino acids Serine 131, Aspartate 177 and Histidine 209. It closely resembles catalytic triads found in other neutral lipases but does not contain a regulatory lid.

The term "variant" or "functionally active variant" of an enzyme as used according to the invention herein means a sequence resulting from modification of the parent sequence by insertion, deletion or substitution of one or more amino acids or nucleotides within the sequence or at either or both of the distal ends of the sequence, and which modification does not affect (in particular impair) the activity of this sequence. In a preferred embodiment the functionally active variant
a) is a biologically active fragment of the amino acid or the nucleotide sequence, the functionally active fragment comprising at least 50% of the sequence of the amino acid or the nucleotide sequence, preferably at least 60%, preferably at least 70%, more preferably at least 80%, still more preferably at least 90%, even more preferably at least 95% and most preferably at least 97%, 98% or 99%;
b) is derived from the amino acid or the nucleotide sequence by at least one amino acid substitution, addition and/or deletion, wherein the functionally active variant has a sequence identity to the amino acid or the nucleotide sequence or to the functionally active fragment as defined in a) of at least 50%, preferably at least 60%, preferably at least 70%, preferably at least 80%, still more preferably at least 90%, even more preferably at least 95% and most preferably at least 97%, 98% or 99%; and/or
c) consists of the amino acid or the nucleotide sequence and additionally at least one amino acid or nucleotide heterologous to the amino acid or the nucleotide sequence, preferably wherein the functionally active variant is derived from or identical to any of the naturally occurring variants of any of the sequences of SEQ ID NO: 1 , 2 and 5 - 12.

"Percent (%) amino acid sequence identity" with respect to the polypeptide sequences identified herein is defined as the percentage of amino acid residues in a candidate sequence that are identical with the amino acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

The functionally active variant may be obtained by sequence alterations in the amino acid or the nucleotide sequence, wherein the sequence alterations retains a function of the unaltered amino acid or the nucleotide sequence, when used in combination of the invention. Such sequence alterations can include, but are not limited to (conservative) substitutions, additions, deletions, mutations and insertions.

In a specific embodiment of the invention the polypeptide or the nucleotide sequence as defined above may be modified by a variety of chemical techniques to produce derivatives having essentially the same activity (as defined above for fragments and variants) as the modified polypeptide or the nucleotide sequence, and optionally having other desirable properties. Other desirable properties are, for example, the increase in thermostability and/or GI stability, as measured by the pH stability and/or protease stability of the enzyme.

The variant of the polypeptide or the nucleotide sequence is functionally active in the context of the present invention, if the activity of the composition of the invention including the variant (but not the original) amounts to at least 50%, preferably at least 60%, more preferred at least 70%, still more preferably at least 80%, especially at least 90%, particularly at least 95%, most preferably at least 99% of the activity of the enzyme as used according to the invention including the amino acid or the nucleotide sequence without sequence alteration (i.e. the original polypeptide or the nucleotide sequence).

Functionally active variants may be obtained by changing the sequence as defined above and are characterized by having a biological activity similar to that displayed by the respective sequence of SEQ ID NO: 1, 2 and 5 - 12 from which the variant is derived, including the ability of ZON hydrolysis in a food product.

In another aspect the functionally active variant of the enzyme as used according to the invention is essentially identical to the amino acid of the SEQ ID NO: 2, 6, 8, 10 or 12, but differs from the amino acid sequence, respectively, in that it is derived from a homologous sequence of a different strain or different species. These are referred to as naturally occurring variants.

Still, the term "functionally active variant" includes naturally occurring allelic variants, as well as mutants or any other non-naturally occurring variants. As is known in the art, an allelic variant is an alternate form of a (poly)peptide that is characterized as having a substitution, deletion, or addition of one or more amino acids that does essentially not alter the biological function of the polypeptide.

In a preferred embodiment, the functionally active variant derived from the amino acid or the nucleotide sequence as defined above by amino acid exchanges, deletions or insertions may also conserve, or more preferably improve, the activity.

Conservative substitutions are those that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains, etc.

Among the well-known mutagenesis approaches for evolving new properties in enzymes are *in vivo* homologous recombination, site-directed mutagenesis, random mutagenesis, error prone PCR and mutagenesis approaches based on the use of genetic engineering methods, such as restriction and ligation. According to a preferred embodiment genes are shuffled enabling the molecular mixing of naturally similar or randomly mutated genes, also called mosaic genes. By using such recombination approaches functionally active variants are preferably provided in a library of variants, which can be used to screen for those variants with improved phenotype. Using mutagenesis allows for the generation of new mutated sequences which code for enzymes with altered, preferably improved functions and/or newly acquired functions. In this way it is possible, for example, to achieve improvements in the thermostability of an enzyme, to change the substrate specificity of an enzyme, to improve its activity, to evolve new catalytic sites and/or to fuse domains from two different enzymes.

In accordance therewith, an enzyme sequence is preferably mutagenized to introduce foreign amino acids into the amino acid sequence, e.g. through mutagenesis methods. The term "foreign" in the context of amino acids shall mean the newly introduced amino acids being naturally occurring, but foreign to the site of modification, or substitutes of naturally occurring amino acids. Mutagenesis methods preferably employ homologous recombination, but random, semi-random or, in particular, by site-directed random mutagenesis methods are also feasible, in particular to delete, exchange or introduce randomly generated inserts into the respective nucleic acid sequences. According to a preferred embodiment sequences of homologous native enzymes are recombined, preferably an N-terminal sequence of one enzyme is combined with a C-terminal sequence of another one with sequence homology, such as a serine hydrolase from another species or another type of serine hydrolases. According to a preferred embodiment the nucleic acid sequences are mutagenized, which are not directly involved in the catalytic center, to improve any characteristics of the enzyme, such as pH or protease stability. However, it is also preferred that sequences of the catalytic center are mutagenized to improve the enzymatic activity.

Particularly preferred are homologous recombination techniques, such as those described in WO03/064667, WO2005/075654.

Therefore homologous DNA sequences are preferred which diverge by at least one nucleotide. In a preferred embodiment of the invention the DNA sequences to be recombined diverge by at least 0.9% up to 35%. Preferably the DNA sequences to be recombined are short sequences with a preferred length in the range of 20 to 3000 nucleotides.

DNA sequences to be recombined may be from natural sources or artificial sequences that are synthetically produced. Natural DNA sequences to be recombined may be derived from any species.

The preferred BTA2 hydrolase variants were produced by homologous recombination with the sequences from the lipase or the PBS A depolymerase. Table 3 provides an overview about preferred recombinant pattern of hits.

A typical library of enzyme variants has a number of enzyme variants of at least 2000 preferably 20000. Using a large library increases the probability of selecting a functionally active variant with improved properties.

Libraries as used according to the invention preferably comprise at least 10² library members, more preferred at least 10³, more preferred at least 10⁴, more preferred at least 10⁵, more preferred at least 10⁶ library members, preferably derived from a parent molecule, which is a functional serine hydrolase as a scaffold to engineer a diverse repertoire of variants which for selecting the best suitable serine hydrolase with improved properties.

The enzyme may be used to detoxify a food product, if there is a risk of ZON contamination. Thus, the food supplement according to the invention is preferably used as a prophylactic measure to avoid unwanted toxic effects, even before determining a ZON concentration in a food product. On the other hand it may be preferred to decontaminate a food product that has a predetermined ZON contamination and could still be used for feeding upon enzymatic treatment.

The *in situ* biotransformation which is effected upon oral administration, i.e. feeding, seems to occur in the small intestine. Therefore, the feed pulp first faces an acid shock in the stomach at a pH 3-5. In the course of digestion in the digestive tract the enzyme activity can then detoxify eventually contaminating mycotoxins before their uptake and metabolism. Animal experiments show that ZON could be degraded immediately upon consumption, i.e. in the stomach.

*Ex vivo* and animal studies are conducted to evaluate the biological effect of the enzymes as used according to the invention.

The term "recombinant" as used herein refers to enzymes produced by recombination techniques employing host cell expression systems, e.g. in a production host cell line, either having the native sequence or variant sequences produced by recombination.

Therefore, nucleotide sequences corresponding to the enzymes according to the invention are also provided. The nucleotide sequences can be used in expression cassettes for transformation of host cells of interest. In particular, expression cassettes for expression of the sequences in plants and other host organisms are provided as well as transformed plants and other host cells.

The term "host cell" or "hosts cell line" refers to a microorganism, used for expression of a recombinant gene to produce the recombinant enzymes as used according to the invention. Preferred host cells are selected from the group consisting of *Escherichia coli, Bacillus spp., Pichia pastoris.*

A host cell clone of cultivated host cells that have proliferated is commonly understood to be a host cell line. A production host cell line is commonly understood to be a cell line ready-to-use for cultivation in a bioreactor to obtain the enzyme product in an industrial scale.

In one aspect, the present invention features a method to mass produce the recombinant enzyme according to the invention in large scale amounts with appropriate purity to enable large scale production for industrial use. In a broad embodiment, the method comprises the step of transfecting an Open Reading Frame encoding for all or part of such an enzyme into a cell suitable for the expression thereof. In some embodiments, a cDNA encoding for a complete enzyme is used. However, in other embodiments, a cDNA encoding for a biologically active variant thereof may be used. Specifically, one or more amino acid substitutions may be made while preserving or enhancing the biological activity of the enzyme. In other preferred embodiments, an expression vector is used to transfer the cDNA into a suitable cell or cell line for expression thereof. In one particularly preferred embodiment, the cDNA is transfected into E. Coli BL21 to create a production cell line. In yet other preferred embodiments, the production procedure features of pH, continuous perfusion or batch fermentation, macroporous microcarriers may be used to produce cell mass followed by a shift to a medium for production in a continuous, fed-batch or batch process to produce the recombinant enzyme on a large scale. Accordingly a yield of at least 1 mg per liter of culture per one production run, or more at peak culturing density can be produced starting with a one liter culture system.

Therefore, the present description also provides for a transfected strain which features the ability to produce the enzyme according to the invention in amounts which enable using the enzyme on an industrial scale. In some preferred embodiments, the strain may contain more than 1 copy of an expression construct. In even more preferred embodiments, the strain expresses the recombinant enzyme in amounts of at least 10 mg/kg biomass.

Expression of the recombinant enzyme according to the invention will also be feasible using yeast, algae, mammalian or plant expression systems, whereby transgenic plants are provided with heterologous genes integrated into the plant genome, such that the resulting plants produce levels of the recombinant enzyme.

In another aspect, the present description provides novel vectors suitable to produce the enzyme according to the invention in amounts which enable using the enzyme on an industrial scale. In preferred embodiments, the present description features an expression vector comprising a promoter/enhancer element or other regulatory elements.

The choice of regulatory sequences, such as promoter and terminator sequences and optionally signal or transit sequences are determined, for example, on the basis of when, where, and how the polypeptide is desired to be expressed. For instance, the expression of the gene encoding a polypeptide of t.

In another aspect, the present description provides for the production of a recombinant enzyme according to the invention. The specific activity of the enzyme according to the present invention is at least 10 mU per milligram protein [One unit is defined as hydrolysis of 1 [mu][eta][iota][omicron][lota] ZON per hour at 37°C and pH 7].

The recombinant enzyme according to the invention preferably is purified by immunoprecipitation, liquid chromatography.

The recombinant enzymes according to the invention are preferably used as a food grade or feed grade material, e.g. obtained upon purification from the host organism.

The foregoing description will be more fully understood with reference to the following examples. Such examples are, however, merely representative of methods of practicing one or more embodiments of the present invention and should not be read as limiting the scope of invention.

### EXAMPLES

### Example 1 : Cell based assays to detect the enzymatic detoxification of ZON

For the screening of a repertoire of enzymes, e.g. a recombination library, a fast and sensitive high throughput assay to detect the degradation of zearalenone was developed.

### Cloning of pERE-SEAP for estrogen sensitive alkaline phosphatase assay in MCF-7;

The Estrogen receptor enhancer from *Xenopus vitellogenin* A2 (Klinge CM (2001) Nucleic Acids Res., 29(14):2905-19.) was cloned into the pTAL-SEAP vector from Clonetech.

pTAL-SEAP was designed for analyzing enhancer sequences by assaying for expression of the secreted alkaline phosphatase (SEAP) gene. Estrogens diffuse into the cell, bind to estrogen receptors (ERs) and activate the transcription of the secreted alkaline phosphatase, regulated by estrogen response elements (EREs). The secreted alkaline phosphatase can easily be detected by adding p-nitrophenylphosphate (pNP) substrate to the culture supernatant and measuring the colorimetric change at OD 405 nm in a 96-well plate reader.

To construct the pERE-SEAP plasmid, the pTAL-SEAP vector was cut with Nhel and Xhol, purified and ligated with the annealed primers encoding for the estrogen receptor enhancer from Xenopus vitellogenin A2 (5' CTA GCG TCA GGT CAC AGT GAC CTG ATC AAA GTT AAT GTA ACC TCA 3'). MCF-7 cells were stably transfected with the pERE-SEAP plasmid, named MCF7 pERE-SEAP pcDNA #1 cell line.

### Final Protocol for the zearalenone degrading MCF-7 ALP Assay:

MCF7 pERE-SEAP pcDNA #1 cells are cultivated in 175 cm² flasks (BD) for one week in estrogen free media (RPMI 1640 without phenol red (PAN Biotech GmbH) supplemented with 10% charcoal stripped FCS (PAA), 1% Pen/Strep (PAN Biotech GH), 1% 10 mM MEM non-essential amino acids (PAN Biotech GmbH)). 2 µl of 10 pM - 10 µM zearalenone solution (10 mM stock solution in DMSO (Applichem), diluted with estrogen free media) is mixed with 10 µl of zearalenone degrading enzyme preparation in 96-wells at 37°C. After 3 h incubation 90 µl of the MCF7 pERE-SEAP pcDNA #1 cell suspension (0.3 x 10⁶ cells/ml estrogen free media) is added to the zearalenone enzyme reaction mix and incubated for 4 days at 37°C.

The alkaline phosphatase secreted into the supernatant of adherent tMCF-7 cells corresponding to the estrogenic activity of zearalenone is measured by the cleavage of p-nitrophenylphosphate substrate: 50 µl of the culture media is transferred into a 96-well plate (BD) and 50 µl of p-nitrophenylphosphate substrate solution (1 mg pNPP in ALP buffer with 200 mM Glycin, 2 mM MgCl₂, 2 mM ZnCl₂, pH 9.6 (Sigma)) is added. After incubation for 1 h at 37°C the amount of the yellow p-nitrophenol is measured in a plate reader (Tecan) at OD 405 nm.

### Example 2: Selection of enzyme candidates for detoxification of zearalenone

Parental enzymes for the recombination were identified which are able to metabolize and detoxify zearalenone. In parallel, high throughput assays were developed for the characterization of the parental enzymes and subsequently the recombinants.

The screening assay of Example 1 makes use of the biological activity of zearalenone as an estradiol-analogue. Commercially available zearalenone (Sigma-Aldrich) is pre-incubated with enzymes of interest, and the mixture is incubated with cell lines susceptible to estrogen treatment (e. g. the MCF7 pERE-SEAP pcDNA #1 cell line). The proliferation of the cells serves as read-out of the assay and helps to identify potential hits. This assay is scaled to high-throughput conditions. Potential hits are further characterized with regards to activity and stability and can be optimized in further rounds of recombination. The leads are then biologically characterized.

Reaction products of ZON were first generated by chemical cleavage of the lactone ring to provide references for the HPLC analysis of enzymatic reactions. After the establishment of the HPLC methods a library of ≥20 different hydrolases was individually incubated with the substrate zearalenone and screened for reaction products by HPLC. The desired detoxification product was ultimately verified by mass spectrometry (MS) analysis.

Zearalenone detoxifying activity was detected for *Thermobifida fusca* BTA- hydrolase 1, an enzyme of the abH25.1 *moxarella* lipase 1 like family.

Other family members homologous to BTA-hydrolase 1 were identified by homology analysis. All enzymes share a high homology in the active centre but have enough diversity over the whole sequence to ensure the generation of a broad diversity by recombination.

**Table 1: Identified zearalenone degrading enzymes:**

| Number | Enzyme | Species |
|---|---|---|
| Myco 021 | Triacylglycerol acylhydrolase | *Streptomyces sp.* |
| Myco 022 | Zearalenone hydrolase (positive control) | *Bionectria ochroleuca* |
| Myco 023 | BTA-hydrolase 2 | *Thermobifida fusca* |
| Myco 024 | Lipase | *Streptomyces exfoliatus* |
| Myco 025 | PBS A depolymerase | *Acidovorax delafieldii* |
| Myco 026 | BTA-hydrolase 1 | *Thermobifida fusca* |

The active center and the catalytic triad are highly conserved in this family of hydrolases, while a prior art zearalenone hydrolase from *Bionectria ochroleuca* (WO03/080842) co-aligned showed significant differences to the enzyme candidates chosen for recombination.

The identity of the enzyme candidates for zearalenone detoxification is in the range of 42 to 92% at the protein level, which is an appropriate range for an in vivo homologous recombination in a mismatch repair deficient recombination system as described in WO03/064667, see Table 2. The lipases from the streptomyces species are the closest relatives, sharing 93% identity, while the PBSA depolymerase from *Acidovorax delafieldii* has the least identical sequence of all four enzyme candidates for recombination (∼62-63 % identity).

**Table 2: Homology of hydrolases. Identity of each pair of enzymes at protein level is listed. Recombined enzymes are highlighted.**

| **Identiy %** | **Myco 021** | **Myco 022** | **Myco 023** | **Myco 024** | **Myco 025** | **Myco 026** |
|---|---|---|---|---|---|---|
| **Myco 021** | 100 % | 16,57 | 57,46 | 89,39 | 43,75 | 57,14 |
| **Myco 022** | | 100 % | 16,42 | 16,03 | 14,08 | 16,47 |
| **Myco 023** | | | 100 % | **56,59** | **43,75** | 92,05 |
| **Myco 024** | | | | 100 % | **42,14** | 56,69 |
| **Myco 025** | | | | | 100 % | 44,20 |
| **Myco 026** | | | | | | 100 % |

All candidate enzymes of Table 1 show ZON degrading activity. Zearalenone hydrolase from Bionectria ochroleuca (Myco 022) was used as positive control.

### Example 3: Generation of library: Recombination of three enzymes with zearalenone detoxifying activity

To generate the screening library the following three genes were chosen as final candidates for the homologous recombination (see US5965415).

Briefly, in a first step the three genes were cloned into the yeast integration plasmid. After digestion and linearization the genes were transformed into the diploid yeast strain. The transformed yeast cells where then spread on selection plates. The heterozygous transformants containing the integration of the linearized plasmid DNA at the wanted genomic position were identified by PCR. The yeast strains were cultured and sporulation was induced.

Between 14 and 16 tetrads of each strain were dissected. Haploids carrying all relevant markers, needed for the generation of the wanted heterozygous diploid strains for the recombination assay, were identified on selection plates.

For the recombination the haploid yeast strains were mated and after induction of sporulation the recombination frequency of the different gene combinations was evaluated.

To transform the recombination library into the final expression plasmids for the high throughput screening, the yeast genomic DNA was isolated from large scale spore preparations grown in corresponding selection medium to be used as template for PCR reactions.

In a two-step procedure, amplicons were generated with oligonucleotides annealing to the flanking marker regions first to select for DNA which had crossovers in between the flanking markers. Second, gene-specific oligonucleotides were used to amplify the recombined genes of the enzymes and to outfit the genes with restriction sites for downstream cloning into expression plasmids.

PCR fragments were cloned into pCR4-TOPO (Invitrogen) and transformed into chemically competent DH5a (NEB) cells. The quality of the six libraries was analyzed by DNA sequencing of single colonies. Each of the six libraries consisted of approximately 2000 - 5000 independent clones (table 3).

**Table 3: Amount of Independent Clones per Library:**

| Library | Independent Clones |
|---|---|
| Myco 023 x Myco 024 | > 2000 |
| Myco 023 x Myco 025 | > 5000 |
| Myco 024 x Myco 023 | > 2000 |
| Myco 024 x Myco 025 | > 5000 |
| Maco 025 x Myco 023 | > 5000 |
| Myco 025 x Myco 024 | > 2000 |

### Example 4: Screening of Recombination Library and Re-Screening of Hits

For the screening of the recombination library the assay of Example 1 is used.

### ZON degrading activity assay

To determine the ZON degrading activity MCF7 pERE-SEAP pcDNA #1 cells were cultivated in 175 cm² flasks for one week in estrogen free media (RPMI 1640 without phenol red (PAN Biotech GmbH) supplemented with 10% charcoal stripped FCS (PAA), 1% Pen/Strep (PAN Biotech GmbH), 1% 10 mM MEM non-essential amino acids (PAN Biotech GmbH)). 2 µl of 10 pM - 10 µl zearalenone solution (Sigma, 10 mM stock solution in DMSO, diluted with estrogen free media) is mixed with 10 µl of zearalenone degrading enzyme preparation. After 3 h incubation at 37°C 90 µl of the MCF7 pERE-SEAP pcDNA #1 cell suspension (0.3 x 10⁶ cells/ml estrogen free media) was added to the zearalenone enzyme reaction mix and incubated in 96-well plates for 4 days at 37°C.

The alkaline phosphatase secreted into the supernatant of adherent tMCF-7 cells corresponding to the estrogenic activity of zearalenone was measured by the cleave of p-nitrophenylphosphate substrate: 50 µl of the culture media was transferred into a 96-well plate and 50 µl of p-nitrophenylphosphate substrate solution (1 mg pNPP in 2xALP buffer with 200 mM Glycerol, 2 mM MgCl₂, 2 mM ZnCl₂, pH 9.6; Sigma) was added. After incubation for 1 h at 37°C the amount of the yellow p-nitrophenol is measured in a plate reader (Tecan) at OD 405 nm.

### p-NP-butyrate Assay:

The p-nitrophenyl-butyrate assay is a fast and convenient assay to measure the expression of the zearalenone hydrolyzing enzymes: 90 µl 0.5 mM pNP-butyrate substrate (Sigma) in 20 mM Tris buffer with 300 mM NaCl pH7 was added to 10 µl of enzyme solution, e.g. cleared lysate. After incubation for 20 min at 37°C the amount of the hydrolyzed p-nitrophenyl-butyrate ester, the yellow p-nitrophenol was measured in a plate reader (Tecan) at OD 405 nm.

### Expression of zonases

The following expression systems were used to express the zonases:
Zonases were expressed intracellularly in either pBAD-HisB (Invitrogen) or pTrcHis2A (Invitrogen). For expression in pBAD-HisB (Invitrogen) the inserts were cut by BamHI and Hindlll (NEB) for ligation with the vector cut by Bgll I and Hindlll (NEB) and transformed into *E*. *coli* Top10 as recommended by Invitrogen. To express the enzymes in pTrcHis2A (Invitrogen), the genes were cloned with and without the signal peptide sequence. pTrcHis2A cut with BamHI and Sail was ligated with the genes which were cut by BamHI and Xhol.

All six libraries were cloned into pBad-His expression-vector. To estimate the number of clones expressing active recombinant enzymes 86 clones of each of the recombination libraries Myco 023 x Myco 024, Myco 024 x Myco 023, Myco 023 x Myco 025, Myco 025 x Myco 023 expressed by pBAD/Top 10 have been tested for hydrolysing activity with pNP-butyrate:
86 clones of each library, 10 wells for controls (TB/Amp only, pBad empty vector, parental enzymes Myco 023, Myco 024 and Myco 022 as positive control) were cultivated in 150 µl TB/Amp overnight, diluted 1:20 into 1 ml TB/Amp in a deep well plate, incubated for 2.5 h at 37°C, induced with 0.2% arabinose and further cultivated for 18 h at 25°C 250 rpm. To lyse the pellet 2 mg/ml lysozyme and a 12-finger sonotrode was used. The lysed cells were centrifuged and cleared lysate was tested in pNP-butyrate assay. As shown in table 4 the number of active clones was dependent on the direction of the parental enzymes.

**Table 4: Percentage of active clones in pNP-butyrate assay:**

| pBAD/Top10 | Myco 023 x Myco 024 | Myco 024 x Myco 023 | Myco 023 x Myco 025 | Myco 025 x Myco 023 |
|---|---|---|---|---|
| | 62% | 85% | 1% | 25% |

### High Throughput Screening

For the high throughput screening of the libraries the enzymes were expressed in 96-deep well plates containing 1 ml culture medium.

Freshly transformed recombination library (comprising 6048 clones plus controls) was grown on LB/Amp agar plates overnight to pick single colonies into 96-well plates with 150 µl TB/Amp/well and incubated at 37°C 220 rpm overnight (controls on each plate: TB/Amp only, pBad empty vector, parental enzymes Myco 023 and Myco 024, Myco 022 as positive control).

The overnight culture was then diluted 1:20 into 1 ml TB/Amp in a 96-deep well plate, and directly induced with 0.2% arabinose, incubated for 2 h at 37°C and further cultivated for 18 h at 25°C 220 rpm. The rest of the overnight culture was supplemented with glycerol and the clones were stored at -80°C.

The cell pellet was lysed after a centrifugation step by adding 200 µl lysis buffer (20 mM Tris, 300 mM NaCl, pH7, with 2 mg/ml lysozyme at 4°C) into the deep wells, resuspended and transferred into a V-shape 96-well plate. A 12-finger sonotrode was used to homogenize the lysate and centrifuged. The cleared lysate was transferred to a new 96-well plate and tested with pNP-butyrate assay for activity at pH 7, stability at pH 3 for 30 min at 37°C and thermostability at 70°C for 30 min.

In a second step the cleared lysate was centrifuged through a 96-well sterile filter plate and 10 µl of the filtrate was added to 2 µl zearalenone dilution. After 3 h incubation 90 µl of the MCF7 pERE-SEAP pcDNA #1 cell suspension (0.3 x 10⁶ cells/ml estrogen free media) was added to the zearalenone enzyme reaction mix and incubated in 96-well plates at 37°C.

After 4 days of incubation 50 µl of the culture media was transferred into a 96-well plate and 50 µl of p-nitrophenylphosphate substrate solution (1 mg pNP in 2xALP buffer with 200 mM glycerol, 2 mM MgCl₂, 2 mM ZnCl₂, pH 9.6) was added. After incubation for 1 h at 37°C the amount of p-nitrophenol was measured in a plate reader (Tecan) at OD 405 nm.

Of the 6048 clones tested in the first screen, 1344 clones were selected for rescreening with the zearalenone/ALP assay. pNP-butyrate assay for activity at pH 7, stability at pH 3 for 30 min at 37°C and thermostability at 70°C for 30 min was repeated. Finally 4 Hits were selected for detailed characterization.

### Purification of hits

Four hits (Myco H03, Myco H04, Myco H10, Myco H11) and the controls (empty vector, parental enzymes Myco 023, Myco 024 and Myco 022 as positive control) were expressed in pTrc His2a in 1 L Terrific Broth media at 37°C 170 rpm in 5 L shake flasks with buffles. After 3 h an OD600 of approximately 2.5 was reached, the temperature was reduced to 25°C and expression was induced by 1 mM IPTG (final concentration). After 17 h the cells were harvested by centrifugation.

The cell pellet (7 - 9 g wet weight) was resuspended in 50 mM phosphate buffer with 300 mM NaCl, 2mg/ml lysozyme, 20µg/ml DNase and protease inhibitor and treated in a microfluidizer to break the cells (3 passages at 1300 bar). The cell lysate was cleared by centrifugation and the enzyme was purified using the Co²⁺ Talon superflow metal affinity matrix (Clonetech).

The cleared lysate was incubated with the matrix overnight at 4°C, washed twice with buffer without imidazole and the bound enzyme was eluted with 150 mM imidazole in 50 mM phosphate buffer with 300 mM NaCl. The buffer was exchanged by gel filtration on PD-10 columns to 20 mM Tris buffer with 300 mM NaCl, pH7, which was more suitable for the MCF-7 cells.

Purification was confirmed by SDS-PAGE, western blot analysis, amido black staining, protein determination and pNP-butyrate assay.

### Thermostability

To measure the thermostability 10 µl purified enzyme (0.5 mg/ml) was mixed with/without 10 µl cleared lysate of *E*. *coli* with empty plasmid and incubated at 50°C, for 5, 15 and 30 minutes. After a centrifugation step at 4000g to remove aggregates, 70 µl 20mM Tris/HCI 300 mM NaCl, pH 7.0 was added and the reaction was started with 10 µl 5 mM pNP-butyrate. Activity was measured at OD 405 nm (37°C) for up to 20 min in a plate reader (Tecan).

As shown in table 5, two of the clones, namely Myco H03 and Myco H11 , demonstrated higher thermostability than reference and parental enzymes retaining full activity after 30 min at 50°C.

**Table 5: Activity after incubation of purified enzymes at 50°C relative to activity after incubation at 4°C.**

| | Relative Activity [%] at 50°C | | |
|---|---|---|---|
| | 5 min | 15 min | 30 min |
| Myco 022 | 33 | 35 | 28 |
| Myco 023 | 100 | 92 | 92 |
| Myco 024 | 16 | 12 | 11 |
| Myco H03 | 98 | 100 | 100 |
| Myco H11 | 86 | 100 | 100 |

### pH Stability

pH stability was measured by mixing 10 µl of purified enzyme (0.5 mg/ml) with 40 µl buffer (pH 3, 4, 5, 6, 7 and 8) and incubated for 30 min at 37°C. The pH was then adjusted to pH 8 by addition of 40 µl 1 M Tris buffer. The reaction was started with 10 µl 5 mM pNP-butyrate and activity was measured at OD 405 nm (37°C) for up to 20 min in a plate reader (Tecan).

As shown in table 6 the enzymes were stable over a broad pH range, with the hits retaining up to 75% residual activity at pH 3 and pH 4. In contrast, the reference enzyme was rapidly inactive at pH 3-5.

**Table 6: Residual activity after incubation for 30 min at specified pH.**

| | Relative Activity [%] | | | | | |
|---|---|---|---|---|---|---|
| | pH 3 | pH 4 | pH 5 | pH 6 | pH 7 | pH 8 |
| Myco 022 | 0 | 17 | 0 | 89 | 100 | 56 |
| Myco 023 | 49 | 48 | 85 | 99 | 100 | 96 |
| Myco 024 | 42 | 21 | 100 | 129 | 100 | 103 |
| Myco H03 | 64 | 61 | 91 | 96 | 100 | 100 |
| Myco H10 | 64 | 57 | 96 | 99 | 100 | 98 |
| Myco H11 | 75 | 73 | 91 | 93 | 100 | 100 |

### Protease Stability

To test protease stability 12.5 µl purified enzyme (0.5 mg/ml) was mixed with or without 12.5 µl cleared lysate of *E. coli* (empty plasmid) and 25 µl pepsin solution (1 mg/ml, 0.1 M HCl, pH 2, Sigma) or pancreatin solution (5 mg/ml 0.1 M NaHC0₃, pH 8.0, Sigma) was added to the enzyme solution and incubated at 37°C. Aggregates were removed by centrifugation at 4000 g and pH was shifted to pH 8 by adding 40 µl 1 M Tris/HCI 300 mM NaCl, pH 8.0. The reaction was started with 10 µl 5 mM pNP-butyrate and activity was measured at OD 405 nm (37°C) for up to 20 min in a plate reader (Tecan).

As shown in table 7 and 8, the parental enzymes and all recombinants were very stable in the presence of pepsin (5 - 30 min) and pancreatin (30 - 120 min) in the presence of cleared lysate from *E*. *coli.* All enzymes were more stable than the reference enzyme Myco 022.

**Table 7: Activity of purified enzymes in cleared lysate of BL21 after incubation with pepsin for 0-30 min.**

| | Relative Activity [%] | | | |
|---|---|---|---|---|
| | 0 min | 5 min | 15 min | 30 min |
| Myco 022 | 100 | 100 | 0 | 0 |
| Myco 023 | 100 | 100 | 100 | 100 |
| Myco 024 | 100 | 100 | 100 | 100 |
| Myco H03 | 100 | 100 | 100 | 100 |
| Myco H04 | 100 | 100 | 100 | 100 |
| Myco H10 | 100 | 100 | 100 | 84 |
| Myco H11 | 100 | 100 | 100 | 98 |

**Table 8: Activity of purified enzymes in cleared lysate of BL21 after incubation with pancreatin for 0 - 120 min.**

| | Relative Activity [%] | | | |
|---|---|---|---|---|
| | 0 min | 30 min | 60 min | 120 min |
| Myco 022 | 100 | 100 | 41 | 68 |
| Myco 023 | 100 | 27 | 36 | 11 |
| Myco 024 | 100 | 100 | 90 | 100 |
| Myco H03 | 100 | 100 | 100 | 100 |
| Myco H04 | 100 | 93 | 100 | 100 |
| Myco H11 | 100 | 100 | 100 | 100 |

## Claims

1. Use of a recombinant zonase having pH stability in the range of pH 3 to 8 and a protease stability in the presence of pancreatic enzymes for treating food products wherein the zonase is any enzyme which is capable of hydrolysing the lactone ring of Zearalenone (ZON) and is selected from the group consisting of hydrolases, lipases, esterases and depolymerases and
wherein the zonase is pH stable if it retains 40 % of residual activity after incubation for 30 min at pH 3 and at 37 °C; and
wherein the protease stability is achieved when the zonase retains either at least 84 % residual activity after incubation of 12,5 µl 0,5 mg/ml purified zonase mixed with 12,5 µl cleared lysate of *E*. *coli* and 25 µl 1 mg/ml pepsin solution (pH 2) for 30 min at 37 °C or at least 27 % residual activity after incubation of 12,5 µl purified zonase solution mixed with 12,5 µl cleared lysate of *E.coli* and 25 µl 5mg/ml pancreatin solution (pH 8) for 30 min at 37 °C,
wherein the zonase is an enzyme of the Moraxella lipase 1 like enzyme family.

2. Use of zonase according to claim 1, wherein the enzyme of the Moraxella lipase 1 like enzyme family is selected from the group of BTA-hydrolase 1 from *Thermobifida fusca,* BTA-hydrolase 2 form *Thermobifida fusca,* lipase from *Streptomyces exfoliates,* PBS A depolymerase from *Acidovoras delafieldii,* and triacylglycerol hydrolase from *Streptomyces sp*., or functionally active variants thereof.

3. Use of a zonase according to claim 1 or 2, wherein the functionally active enzyme variant with zonase activity is obtained by *in* vivo recombination of at least two parentals selected from the group consisting of BTA-hydrolase 1 from *Thermobifida fusca,* BTA-hydrolase 2 from *Thermobifida fusca,* lipase from *Streptomyces exfoliatus,* PBS A depolymerase from *Acidovoras delafieldii,* and triacylglycerol hydrolase from *Streptomyces sp.*

4. Use of a zonase according to claim 3, wherein one parental is BTA-hydrolase 2.

5. Use of a zonase according to claim 4, comprising at least 25 consecutive amino acids selected from the amino acid sequence set forth in SEQ ID NO: 6 and at least 25 consecutive amino acids selected from the amino acid sequence as set forth in SEQ ID NO: 8 or SEQ ID NO: 10.

6. Use of zonase according to claim 4, wherein the zonase is selected from the group of consisting of the amino acid sequence as set forth in SEQ ID NO: 13, 14, 15 and 16.

7. A food supplement for treating a potentially zearalenone contaminated food product comprising an enzyme product containing a zonase according to any of claims 1 to 6.

8. The food supplement according to claim 7, wherein the enzyme has a zearalenone (ZON) detoxification activity in a cell based assay of at least 40 mU/mg enzyme, wherein one unit (U) is defined as hydrolysis of 1 µmol ZON per hour at 37 °C and pH 7.

9. A feed product comprising grain and at least one recombinant zonase according to any of claims 1 to 6.

10. The feed product according to claim 9, which contains the enzyme in a dry mixture.

11. The feed product according to claim 9 or 10, wherein the grain is selected from corn, wheat, barley, rye, rice, sorghum and millet.

## Patentansprüche

1. Verwendung einer rekombinanten Zonase, die eine pH-Stabilität in dem Bereich von pH 3 bis 8 aufweist und eine Proteasestabilität in der Anwesenheit von Pankreasenzymen aufweist, zum Behandeln von Nahrungsmitteln, wobei die Zonase irgendein Enzym ist, welches fähig ist, den Laktonring von Zearalenon (ZONE) zu hydrolysieren und gewählt ist aus der Gruppe bestehend aus Hydrolasen, Lipasen, Esterasen und Depolymerasen, und
worin die Zonase pH-stabil ist, wenn sie 40 % Restaktivität nach Inkubation für 30 min. bei pH 3 und 37 °C aufweist, und
worin die Proteasestabilität erreicht wird, wenn die Zonase entweder wenigstens 84 % Restaktivität nach Inkubation von 12,5 µl 0,5 mg/ml gereinigter Zonase gemischt mit 12,5 µl geklärtem *E*. *coli*-Lysat und 25 µl 1 mg/ml Pepsinlösung (pH 2) für 30 min. bei 37 °C oder wenigstens 27 % Restaktivität nach Inkubation von 12,5 µl gereinigter Zonaselösung gemischt mit 12,5 µl geklärtem *E*. *coli*-Lysat und 25 µl 5 mg/ml Pankreatinlösung (pH 8) für 30 min. bei 37 °C aufweist,
worin die Zonase ein Enzym der Moraxella-Lipase 1 artigen Enzymfamilie ist.

2. Verwendung einer Zonase gemäß Anspruch 1, worin das Enzym der Maraxella-Lipase 1 artigen Enzymfamilie aus der Gruppe von BTA-Hydrolase 1 von *Thermobifida fusca,* BTA-Hydrolyse 2 von *Thermobifida fusca,* Lipase von *Streptomyces exfoliates,* PBS A Depolymerase von Acidovoras *delafieldii* und Triacylglycerol-Hydrolase von *Streptomyces sp*. oder funktionell aktiven Varianten davon gewählt ist.

3. Verwendung einer Zonase nach Anspruch 1 oder 2, worin die funktionell aktive Enzymvariante mit Zonaseaktivität durch *in vivo* Rekombination von wenigstens 2 Eltern gewählt aus der Gruppe bestehend aus BTA-Hydrolase 1 von *Thermobifida fusca,* BTA-Hydrolase 2 von *Termobifida fusca,* Lipase von *Streptomyces exfoliatus,* PBS A Depolymerase aus *Acidovoras delafieldii* und Triacylglycerol-Hydrolyse aus *Streptomyces sp.* erhalten wird.

4. Verwendung einer Zonase nach Anspruch 3, worin ein Elternteil BTA-Hydrolase 2 ist.

5. Verwendung einer Zonase nach Anspruch 4, umfassend wenigstens 25 aufeinanderfolgende Aminosäuren gewählt aus der Aminosäuresequenz, die in SEQ ID Nr. 6 festgelegt ist und wenigstens 25 aufeinanderfolgende Aminosäuren gewählt aus der Aminosäuresequenz, wie sie in SEQ ID Nr. 8 oder SEQ ID Nr. 10 festgelegt ist.

6. Verwendung einer Zonase nach Anspruch 4, worin die Zonase aus der Gruppe gewählt ist, bestehend aus der Aminosäuresequenz, wie sie in SEQ ID Nrn. 13, 14, 15 und 16 festgelegt ist.

7. Nahrungsergänzung bzw. Futtermittelergänzung zur Behandlung eines potentiellen Zearalenon-kontaminierten Lebensmittelprodukts, umfassend ein Enzymprodukt enthaltend eine Zonase gemäß einem der Ansprüche 1 bis 6.

8. Nahrungsmittelzusatz nach Anspruch 7, worin das Enzym eine Zearalenon- (ZON-) detoxifizierende Wirkung in einem zellbasierten Versuch von wenigstens 40 mU/mg Enzym aufweist, wobei eine Einheit (U) als eine Hydrolyse von 1 µmol ZON pro Stunde bei 37 °C und pH 7 definiert ist.

9. Nahrungsmittelprodukt, umfassend Getreide und wenigstens eine rekombinante Zonase gemäß einem der Ansprüche 1 bis 6.

10. Nahrungsmittelprodukt nach Anspruch 9, welches das Enzym in einer Trockenmischung enthält.

11. Nahrungsmittelprodukt nach Anspruch 9 oder 10, worin das Getreide aus Weizen, Gerste, Roggen, Reis, Sorghum und Hirse gewählt ist.

## Revendications

1. Utilisation d'une zonase recombinante ayant une stabilité en pH dans la plage de pH 3 à 8 et une stabilité de protéase en présence d'enzymes pancréatiques pour traiter des produits alimentaires, dans laquelle la zonase est toute enzyme qui est capable d'hydrolyser le cycle lactone de la zéaralénone (ZON) et est choisie dans le groupe consistant en les hydrolases, les lipases, les estérases et les dépolymérases et
dans laquelle la zonase est stable en pH si elle conserve 40 % d'activité résiduelle après incubation pendant 30 min à pH 3 et à 37 °C ; et
dans laquelle la stabilité de protéase est accomplie lorsque la zonase conserve soit au moins 84 % d'activité résiduelle après incubation de 12,5 µl de zonase purifiée à 0,5 mg/ml mélangée avec 12,5 µl de lysat clarifié de *E. coli* et 25 µl de solution de pepsine à 1 mg/ml (pH 2) pendant 30 min à 37 °C ou au moins 27 % d'activité résiduelle après incubation de 12,5 µl de solution de zonase purifiée mélangée avec 12,5 µl de lysat clarifié de *E. coli* et 25 µl de solution de pancréatine à 5 mg/ml (pH 8) pendant 30 min à 37 °C,
dans laquelle la zonase est une enzyme de la famille d'enzyme de type lipase 1 de Moraxella.

2. Utilisation de zonase selon la revendication 1, dans laquelle l'enzyme de la famille d'enzyme de type lipase 1 de Moraxella est choisie dans le groupe de BTA-hydrolase 1 provenant de *Thermobifida fusca,* BTA-hydrolase 2 provenant de *Thermobifida fusca,* lipase provenant d'exfoliats de *Streptomyces,* PBS A dépolymérase provenant d'*Acidovoras delafieldii,* et de triacyclycéroyl hydrolase provenant de *Streptomyces sp*., ou de variants fonctionnellement actifs de celles-ci.

3. Utilisation d'une zonase selon la revendication 1 ou 2, dans laquelle le variant d'enzyme fonctionnellement actif avec activité de zonase est obtenu par une recombinaison *in vivo* d'au moins deux parents choisis dans le groupe consistant en la BTA-hydrolase 1 provenant de *Thermobifida fusca,* la BTA-hydrolase 2 provenant de *Thermobifida fusca,* la lipase provenant de *Streptomyces exfoliatus,* la PBS A dépolymérase provenant d'*Acidovoras delafieldii,* et la triacylglycérol hydrolase provenant de *Streptomyces sp.*

4. Utilisation d'une zonase selon la revendication 3, dans laquelle un parent est la BTA-hydrolase 2.

5. Utilisation d'une zonase selon la revendication 4, comprenant au moins 25 acides aminés consécutifs choisis dans la séquence d'acides aminés précisée dans SEQ ID N° 6 et au moins 25 acides aminés consécutifs choisis parmi la séquence d'acides aminés telle que précisée dans SEQ ID N° 8 ou SEQ ID N° 10.

6. Utilisation d'une zonase selon la revendication 4, dans laquelle la zonase est choisie dans le groupe consistant en la séquence d'acides aminés telle que précisée dans SEQ ID N° 13, 14, 15 et 16.

7. Complément alimentaire destiné à traiter un produit alimentaire potentiellement contaminé par de la zéaralénone comprenant un produit d'enzyme contenant une zonase selon l'une quelconque des revendications 1 à 6.

8. Complément alimentaire selon la revendication 7, dans lequel l'enzyme a une activité de détoxification de zéaralénone (ZON) dans un dosage sur cellules d'au moins 40 mU/mg d'enzyme, dans lequel une unité (U) est définie comme l'hydrolyse de 1 µmole de ZON par heure à 37 °C et pH 7.

9. Produit alimentaire comprenant des céréales et au moins une zonase recombinante selon l'une quelconque des revendications 1 à 6.

10. Produit alimentaire selon la revendication 9, qui contient l'enzyme dans un mélange sec.

11. Produit alimentaire selon la revendication 9 ou 10, dans lequel les céréales sont choisies parmi le maïs, le blé, l'orge, le seigle, le riz, le sorgho et le millet.
